# EUROPEAN PATENT SPECIFICATION

(11) **EP 4 003 396 B1**
(45) Date of publication and mention of the grant of the patent: **19.03.2025**
(21) Application number: 20734889.7
(22) Date of filing: 23.06.2020
(51) Int. Cl.: A61K 38/17, A61K 9/00, A61K 31/4725, A61K 31/573, A61K 38/13, A61P 27/02, A61P 27/04

(54) **TSG6 POLYPEPTIDE FRAGMENT FOR DRY EYE DISEASE**
TSG6-POLYPEPTIDFRAGMENT FÜR TROCKENES AUGE
FRAGMENT POLYPEPTIDIQUE DE TSG6 POUR SYNDROME DE L'OEIL SEC

(30) Priority: 25.07.2019 GB 201910645
(43) Date of publication of application: 01.06.2022
(73) Proprietor: The University of Manchester, Manchester, Greater Manchester M13 9PL (GB)
(72) Inventor: DAY, Anthony, Manchester Greater Manchester M13 9PL (GB); MILNER, Caroline, Manchester Greater Manchester M13 9PL (GB); OH, Joo Youn, Seoul 03080 (KR)
(74) Representative: Mewburn Ellis LLP
(86) International application number: PCT/EP2020/067448
(87) International publication number: WO 2021/013452

(56) References cited:
- US-A1- 2015 057 229
- US-A1- 2016 075 750
- YU JEONG KIM ET AL: "Comparison of Topical Application of TSG-6, Cyclosporine, and Prednisolone for Treating Dry Eye :", CORNEA: THE JOURNAL OF CORNEA AND EXTERNAL DISEASE, vol. 35, no. 4, 1 April 2016 (2016-04-01), US, pages 536 - 542, XP055729831, ISSN: 0277-3740, DOI: 10.1097/ICO.0000000000000756

## Description

### Field of the Invention

The present invention relates to the treatment of ocular surface disorders and particularly, although not exclusively, to the treatment of dry eye disease with a LINK_TSG6 polypeptide.

### Background

Tumour necrosis factor (TNF)-stimulated gene 6 (TSG-6) is a ~35 kDa secreted product of TNF-stimulated gene-6, expressed in response to inflammatory mediators and growth factors.

While constitutively expressed in a few tissues, TSG-6 is generally upregulated wherever there is inflammation. For the most part TSG-6 exhibits anti-inflammatory and tissue protective properties, but has been implicated as sometimes playing a role in disease pathology, for example, in the lung. While being made by a broad range of cell types, it was the finding that TSG-6 is produced by mesenchymal stem/stromal cells (MSCs) in response to inflammatory signals, and that it mediates many of their immunomodulatory and reparative activities, which has led to a wealth of publications on the therapeutic effects of this intriguing molecule across a wide range of disease models.

TSG-6 is a relatively small protein, with a molecular mass of only ~35-38 kDa, being mainly composed of two modular domains. Given TSG-6's size, it has a surprisingly large number of activities, including the modulation of immune and stromal cell function and its contribution to extracellular matrix formation, mechanics and remodelling. It is the ability of TSG-6 to regulate matrix organization, and to control the association of matrix molecules with cell surface receptors and with extracellular signalling factors (e.g. chemokines), that likely underlies its diverse functional repertoire. In this regard, TSG-6 interacts with a large array of ligands, such as glycosaminoglycans (GAGs), proteoglycan (PG) core proteins and other matrix components, and binds directly to multiple chemokines and bone morphogenetic proteins (BMPs). One particularly unusual function of TSG-6 is its role as an enzyme that catalyses the covalent modification of the non-sulfated GAG hyaluronan (HA) with so-called heavy chains (HCs) from the inter-α-inhibitor (IαI) family of proteoglycans. This process, mediated by the full-length TSG6 protein, but not LINK_TSG6 polypeptides containing only a fragment of TSG-6, results in the formation of HC•HA complexes, and is essential for mammalian ovulation and fertilisation, and also occurs in many other contexts (e.g. inflammation) where HC•HAs either confer tissue protection or contribute to pathological processes.

The sites and contexts of TSG-6 expression, its structure and ligand-binding properties, and how these together underpin its diverse biology and therapeutic potential at a molecular level are reviewed in Day & Milner (Matrix Biology (2019) 78-79, 60-83).

US2015/0057229 describes the use of LINK_TSG6 in inhibiting cartilage degradation.

Dry eye disease (also known as keratoconjunctivitis sicca) is one of the most common ocular diseases, occurring in between 7% and 33% of the population worldwide. It is a multifactorial condition of the tear film and ocular surface and is accompanied by increased osmolarity of the tear film and inflammation of the ocular surface, neurotrophic deficiency and meibomian gland dysfunction.

Kim et al. (2016) (Cornea 35(4), 536-542) compared topically applied TSG-6, cyclosporine and prednisolone for treating dry eye. 12 week old NOD.B10.H2b mice were topically administered with recombinant TSG-6 (0.1%) 4 times a day, 0.05% cyclosporine (Restasis) twice a day, or 1% prednisolone (Pred Forte) 4 times a day for 1 week. Topical TSG-6 was found to be as effective in inflammation mediated dry eye as cyclosporine eye drops. However, they conclude that clinical application of TSG-6 is limited by various factors, including difficulty in large-scale production or variation in stability of the recombinant protein.

WO2011/139357 described the use of adult stem cells/progenitor cells and stem cell proteins for the treatment of eye injuries and diseases. They propose therapy based on the discovery that after a chemical burn to the cornea of a rat, application of MSCs or MSC conditioned medium reduced inflammation and revascularisation. They proposed the use of anti-apoptotic and anti-inflammatory proteins such as STC-1 and TSG-6, which are expressed by mesenchymal stem cells. Corneal surface inflammation was created in rat eyes by ethanol application and mechanical debridement of the corneal and limbal epithelium. Application of recombinant full length TSG-6 resulted in reduced corneal opacity and neovascularization as compared to a PBS control, and the authors conclude that proteins produced by MSCs in response to an injury signal can protect the corneal surface from damage by increasing the viability and proliferation of corneal epithelial progenitors and by suppressing inflammation at the corneal surface.

US2016/0075750 (Prockop et al) described a method of producing a protein or polypeptide, such as TSG-6 protein, in mammalian cells suspended in a protein-free medium that includes at least one agent that suppresses production of hyaluronic acid, hyaluronan or a salt thereof.

The present invention has been devised in light of the above considerations.

### Summary of the Invention

The invention is defined in the claims.

The present disclosure relates to LINK_TSG6 polypeptide for use in the treatment or prevention of ocular surface disorder such as dry eye disease or other ocular surface disorders with corneal lesions similar to those observed in dry eye disease. The inventors have found that this polypeptide is capable of reducing or preventing the signs and symptoms of dry eye disease, in a dose-dependent manner. Surprisingly, the inventors have found that this polypeptide is more potent at reducing corneal epithelial defects than full length TSG-6.

In some cases, the treatment or prevention of dry eye disease described herein comprises one or more effects selected from the group consisting: healing of corneal epithelial defects; increase in tear production; suppression of inflammation; and an increase in, or the preservation of, the number of conjunctival goblet cells. Suppression of inflammation may comprise a decrease in the production of one or more pro-inflammatory cytokines in the cornea, the intraorbital lacrimal glands, or both the cornea and the intraorbital lacrimal glands, the pro-inflammatory cytokines optionally selected from TNFα, IFNγ, IL-6 and IL-1β.

The treatment or prevention of dry eye disease may comprise healing of corneal epithelial defects, increase in tear production, suppression of inflammation or an increase in, or the preservation of the number of conjunctival goblet cells as compared to the corneal epithelial defects, tear production, inflammation or number of conjunctival goblet cells prior to the administration of LINK_TSG6 polypeptide, and as compared to administration of PBS vehicle.

The treatment or prevention of dry eye disease may comprise healing of corneal epithelial defects, increase in tear production, suppression of inflammation or an increase in, or the preservation of the number of conjunctival goblet cells as compared to the corneal epithelial defects, tear production, inflammation or number of conjunctival goblet cells in a control individual treated with full-length TSG-6 protein.

In some aspects described herein, the treatment comprises topical administration of LINK_TSG6 polypeptide to the eye. The LINK_TSG6 polypeptide may be formulated as an eye drop. The treatment may comprise the administration of an eye drop comprising LINK_TSG6. The LINK_TSG6 polypeptide may be formulated with, or the treatment may involve co-administration with prednisolone, cyclosporine, Lifitegrast (Xiidra^{™}), artificial tears, or any combination thereof.

In some aspects, the treatment comprises administering LINK_TSG6 polypeptide two times per day. In some aspects, the treatment comprises administering LINK_TSG6 polypeptide more than two times per day. In some cases, the treatment comprises administering LINK_TSG6 polypeptide fewer than 4 times per day, or fewer than 3 times per day. In some cases, the treatment comprises administering LINK_TSG6 polypeptide once per day. In some cases, the treatment comprises administering LINK_TSG6 polypeptide less frequently than once per day, such as once every two days, one time every three days, once every week, or once every two weeks.

The treatment may involve the administration of between 10-200 µg LINK_TSG6 per eye, such as between 100 and 200 µg LINK_TSG6 per eye, between 100 and 150 µg LINK_TSG6 per eye, between 120 and 150 µg LINK_TSG6 per eye. Preferably, the treatment involves the administration of around 120µg-150µg LINK_TSG6 polypeptide per eye, or around 12-15 µg LINK_TSG6 per eye.

The treatment or prevention of ocular surface disorders such as dry eye disease disclosed herein is applicable to dry eye disease associated with any cause. The individual being treated may have a condition that is associated with an increased incidence of dry eye disease, such as Sjögren's syndrome, rheumatoid arthritis or diabetes. In some cases, the individual has Type 1 diabetes or Type 2 diabetes. The individual may be at risk of developing an ocular surface disorder such as dry eye disease due to aging, exposure to air pollution and/or increased use of a visual device (e.g. smart phone, computer, tablet).

The treatment involves the administration of LINK_TSG6 polypeptide that consists of (i) the amino acid sequence of SEQ ID NO: 7 or SEQ ID NO: 9, or (ii) an amino acid sequence having at least 80% identity to the amino acid sequence of SEQ ID NO: 7 or 9.

A further aspect disclosed herein is a pharmaceutical composition comprising LINK_TSG6 polypeptide. The pharmaceutical composition may comprise LINK_TSG6 polypeptide solubilised in saline. The pharmaceutical composition may comprise LINK_TSG6 polypeptide solubilised in phosphate buffered saline. In some aspects, the pharmaceutical composition comprises at least 2000 µg/ml, 2100 µg/ml, 2200 µg/ml, 2300 µg/ml 2400µg/ml, 2500 µg/ml, 2600 µg/ml, 2700 µg/ml, 2800 µg/ml, 2900 µg/ml, 3000 µg/ml, 3100 µg/ml, 3200 µg/ml, 3300 µg/ml or more than 3300 µg/ml LINK_TSG6. In some aspects, the pharmaceutical composition comprises at least 200 µg/ml, 300 µg/ml, 400 µg/ml, 500 µg/ml, 600 µg/ml, 700 µg/ml, 800 µg/ml, 900 µg/ml, 1000 µg/ml, 1200 µg/ml, 1300 µg/ml, 1400 µg/ml, 1500 µg/ml, 1600 µg/ml, 1700 µg/ml, 1800 µg/ml 1900 µg/ml, 2000 µg/ml LINK_TSG6. In some aspects, the pharmaceutical composition comprises at least 200 µg/ml, 300 µg/ml, 400 µg/ml, 500 µg/ml, 600 µg/ml, 700 µg/ml, 800 µg/ml, 900 µg/ml, 1000 µg/ml, 1200 µg/ml, 1300 µg/ml, 1400 µg/ml, 1500 µg/ml, 1600 µg/ml, 1700 µg/ml, 1800 µg/ml 1900 µg/ml, 2000 µg/ml LINK_TSG6. Preferably, the pharmaceutical formation comprises at least 2000 µg/ml LINK_TSG6. Such formulations are useful for delivering between around 120 µg and around 150µg of LINK_TSG6 per drop.

The pharmaceutical composition may be an eye drop formulation. The eye drop formulation may comprise between 1500 µg/ml and 3500 µg/ml, between 1500 µg/ml and 3000 µg/ml, between 2000 µg/ml and 3000 µg/ml, or between 2400 µg/ml and 3000 µg/ml. Preferably, the eye drop formulation comprises between about 2400 µg/ml and about 3000 µg/ml. In some cases, the eye drop formulation comprises at least 2200, at least 2300 µg/ml, at least 2400 µg/ml, at least 2500 µg/ml, at least 2600 µg/ml, at least 2700 µg/ml, at least 2800 µg/ml, at least 2900 µg/ml, at least 3000 µg/ml or more than 3000 µg/ml. The eye drop formulation may further comprise prednisolone, cyclosporine, Lifitegrast (Xiidra^{™}) or artificial tears. The eye drop formulation may comprise a pharmaceutically acceptable carrier. The eye drop formulation may comprise LINK_TSG6 polypeptide solubilised in saline. The eye drop formulation may comprise LINK_TSG6 polypeptide solubilised in phosphate buffered saline.

The disclosure includes the combination of the aspects and preferred features described except where such a combination is clearly impermissible or expressly avoided.

### Summary of the Figures

Aspects and experiments illustrating the principles of the disclosure will now be discussed with reference to the accompanying figures in which:

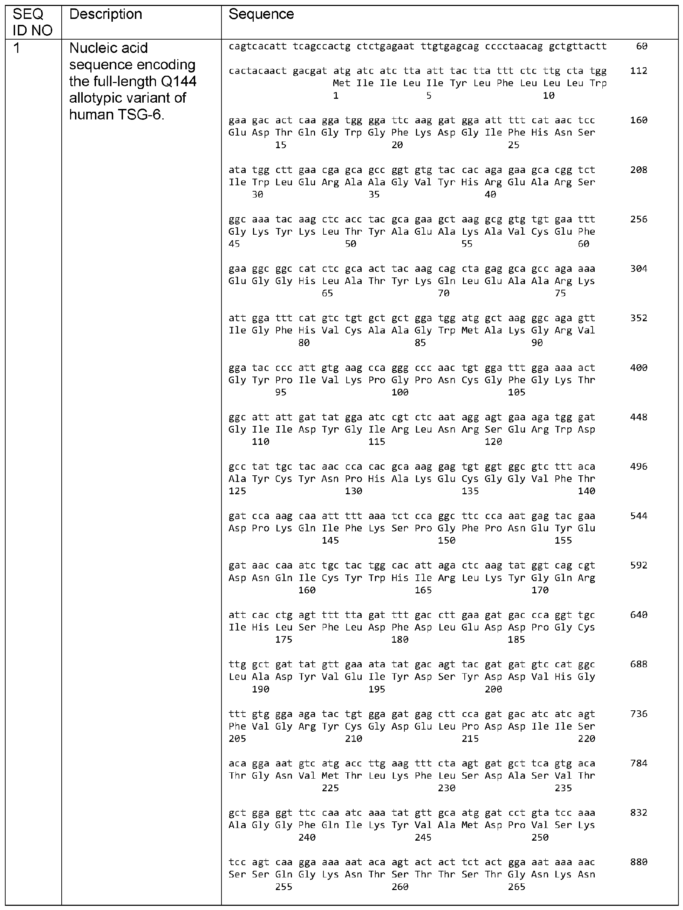

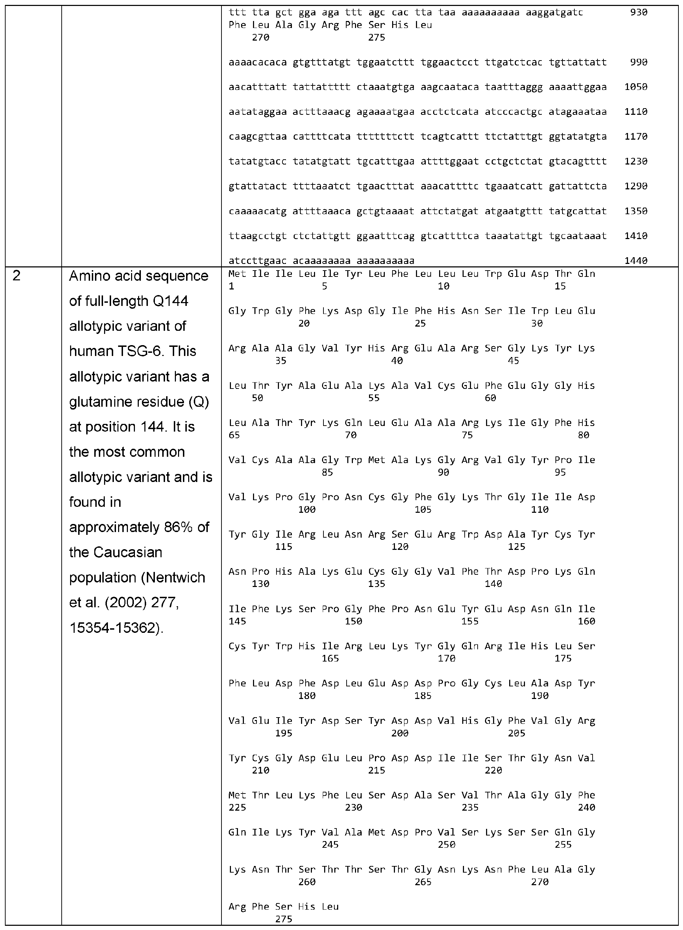

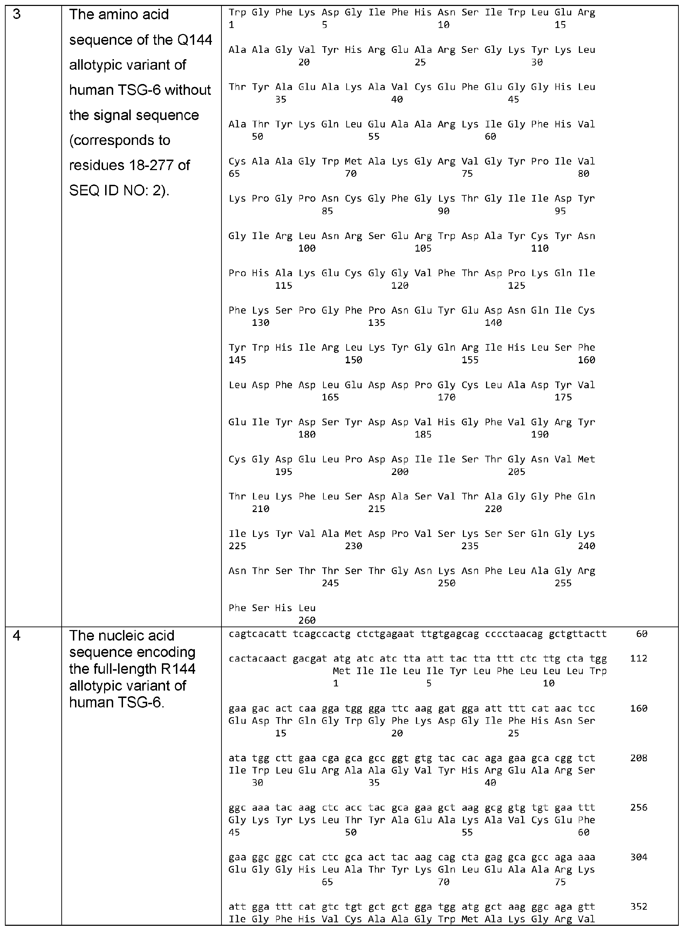

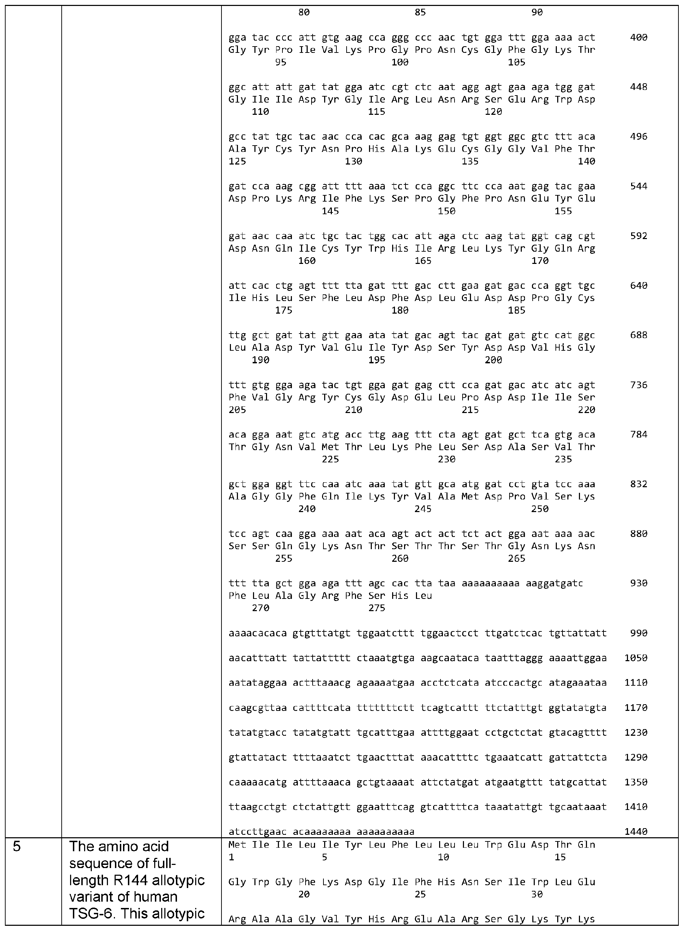

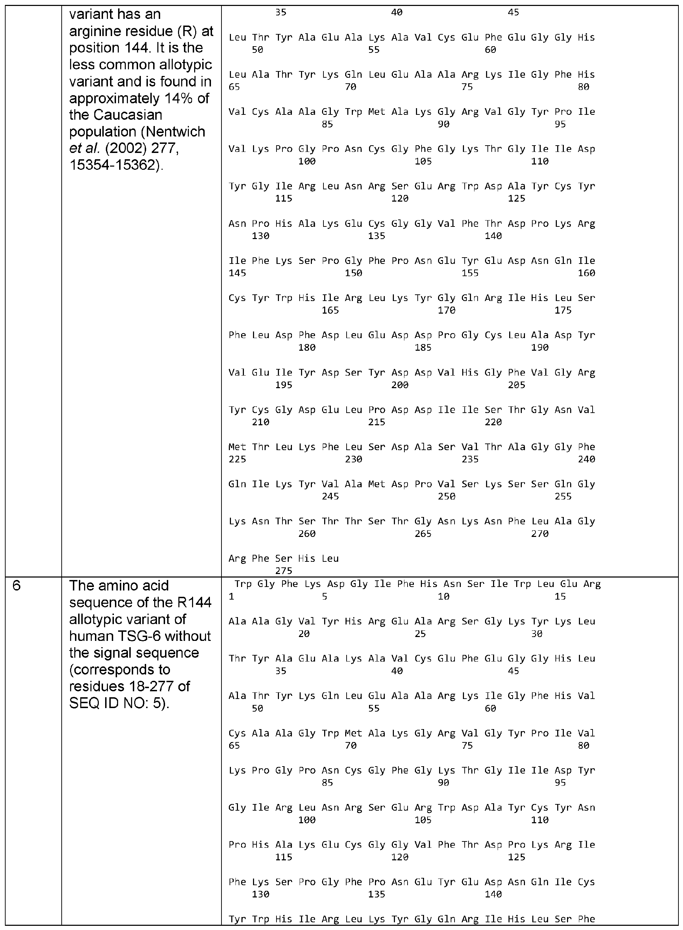

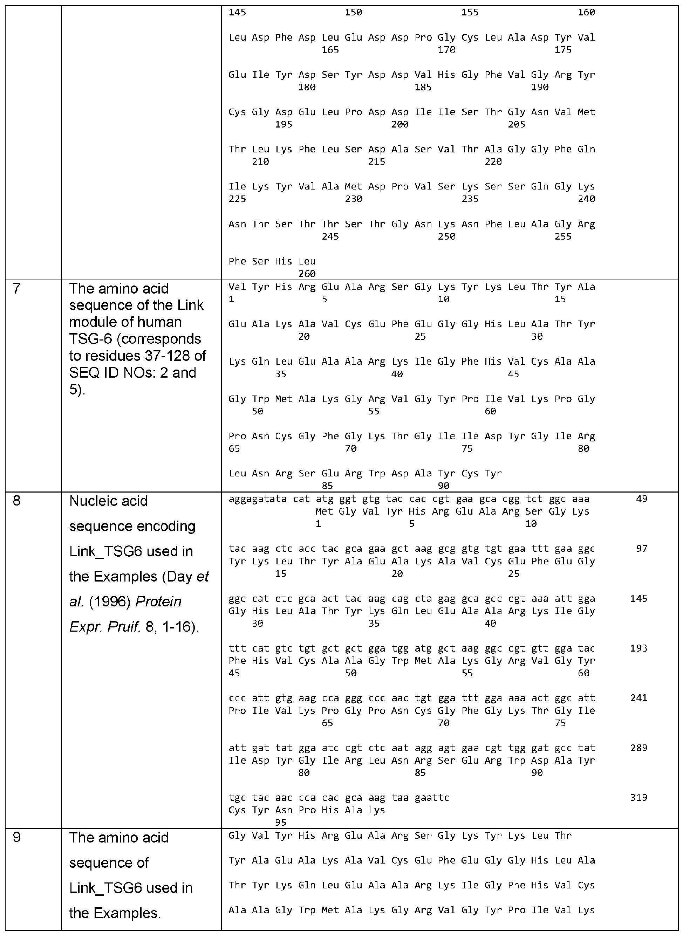

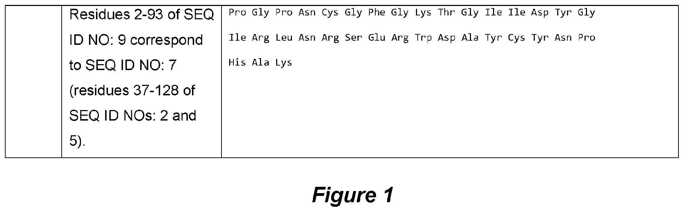

**Figure 1****.** Sequences relevant to the present disclosure.

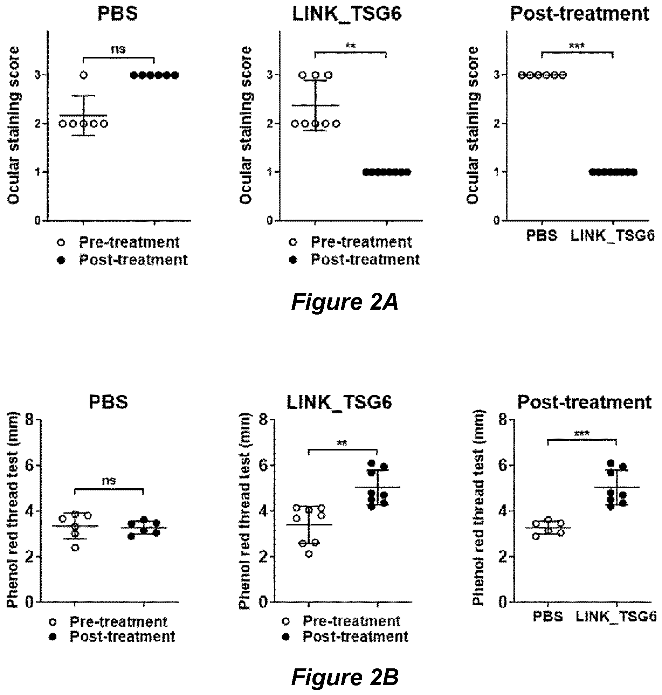

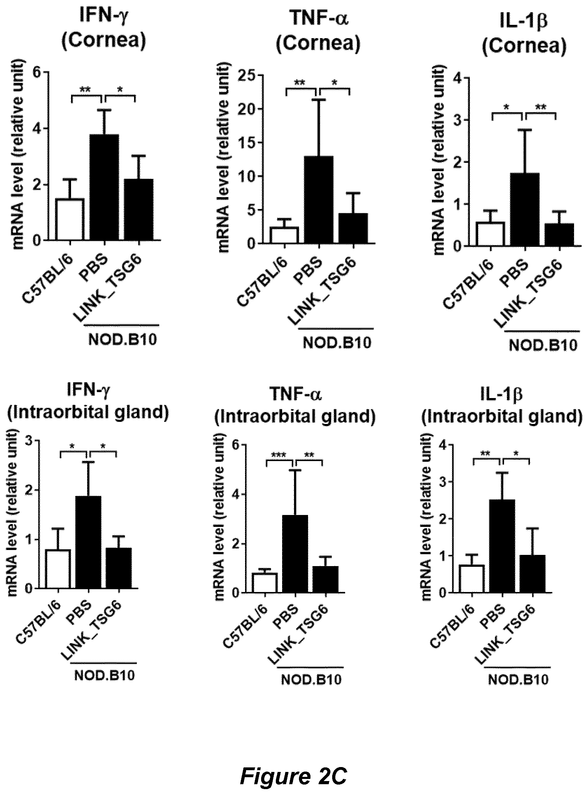

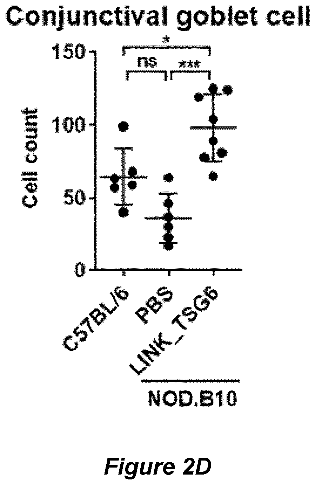

**Figure 2****.** LINK_TSG6 reduces the signs of dry eye disease. A. Ocular staining score following lissamine green staining; B. Aqueous tear production as determined by phenol red thread test; C. Pro-inflammatory cytokine mRNA level as determined by real-time RT-PCR; D. Conjunctival goblet cell counts in PAS (Periodic acid-Schiff stain) stained conjunctival sections. For A and B significance was determined by Wilcoxon matched-pairs signed rank test for comparison between pre- and post-treatment and Mann-Whitney U test for comparison between PBS and LINK_TSG6. ns = not significant, p>0.05; ** = p<0.01; *** = p<0.001. For C and D significance was determined by one-way ANOVA and Tukey's multiple comparisons test. ns = not significant, p>0.05; * = p<0.05; ** = p<0.01; *** = p<0.001; **** = p<0.0001.

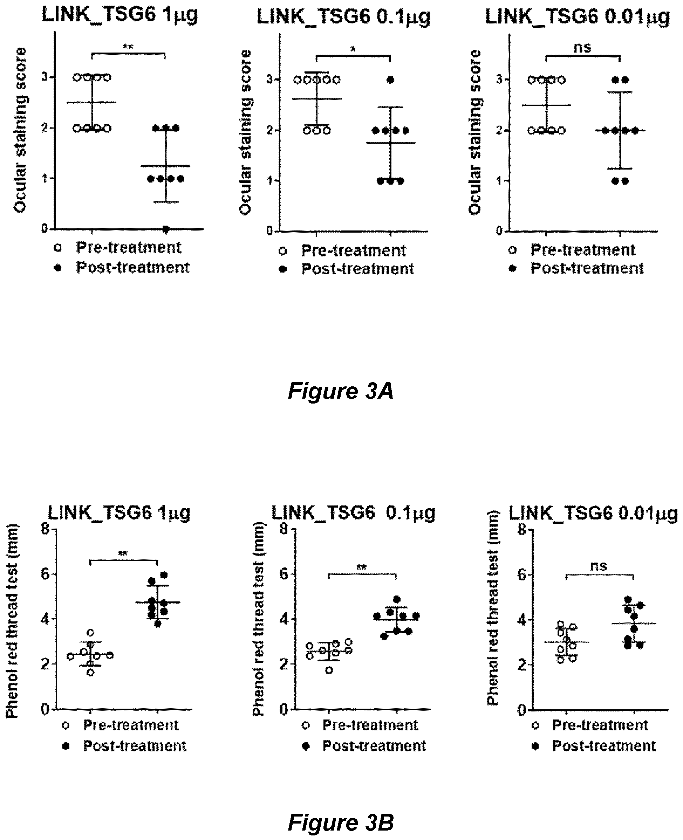

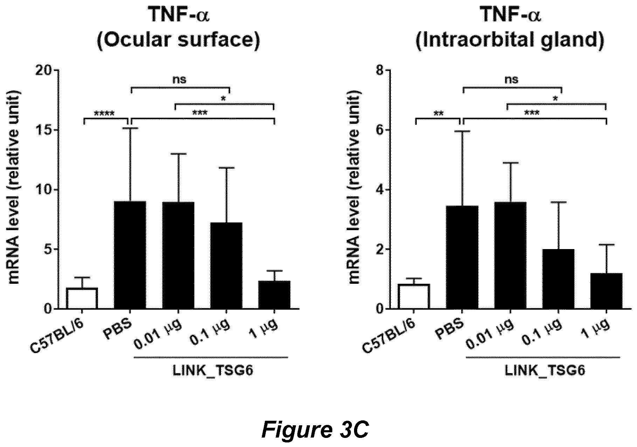

**Figure 3****.** LINK_TSG6 reduces the signs of dry eye disease in a dose-dependent manner. A. Quantitation of corneal epithelial defects from lissamine green-stained corneas. LINK_TSG6 1 µg and 0.1 µg was effective in reducing corneal epithelial defects, but LINK_TSG6 0.01 µg did not significantly improve corneal epithelial defects. B. Quantification of aqueous tear production by phenol red thread test. LINK_TSG6 1 µg and 0.1 µg were both effective in increasing the amount of tear production, but LINK_TSG6 0.01 µg did not significantly improve tear production. C. Quantification of pro-inflammatory cytokines by real-time RT-PCR analysis. LINK_TSG6 1 µg was most effective in suppressing TNF-α expression. In A and B significance was determined by Wilcoxon matched-pairs signed rank test. * = p<0.05; ** = p<0.01; ns = not significant, p>0.05. In C significance was determined by one-way ANOVA and Tukey's multiple comparisons test. ns = not significant, p>0.05; * = p<0.05; ** = p<0.01 *** = p<0.001; **** = p<0.0001.

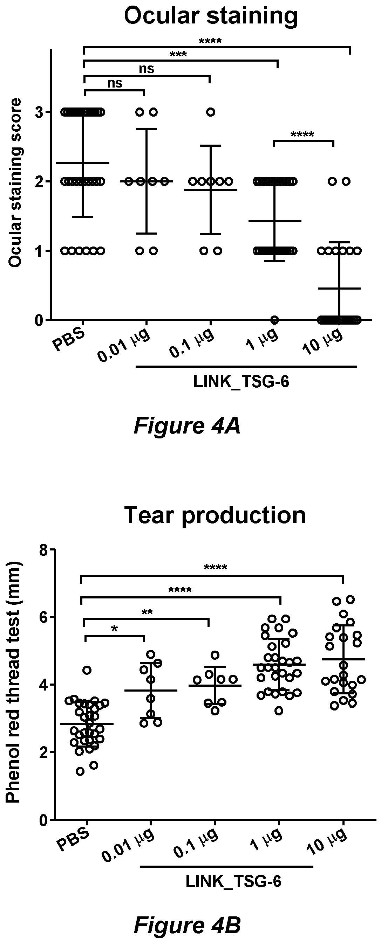

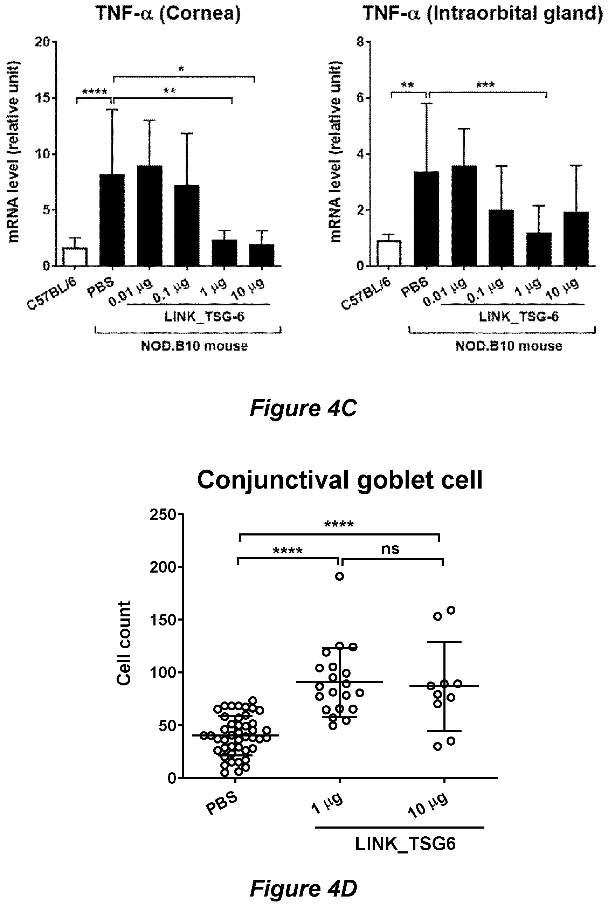

**Figure 4****.** Dose response data for LINK_TSG6. A. Quantitation of corneal epithelial defects from lissamine green-stained corneas by ocular staining; B. Quantification of aqueous tear production by phenol red thread test; C. Pro-inflammatory cytokine mRNA level as determined by real-time RT-PCR D. Conjunctival goblet cell counts in PAS stained conjunctival sections. Significance determined by one-way ANOVA and Tukey's multiple comparisons tests. ns = not significant, p>0.05; * = p<0.05; ** = p<0.01 *** = p<0.001; **** = p<0.0001.

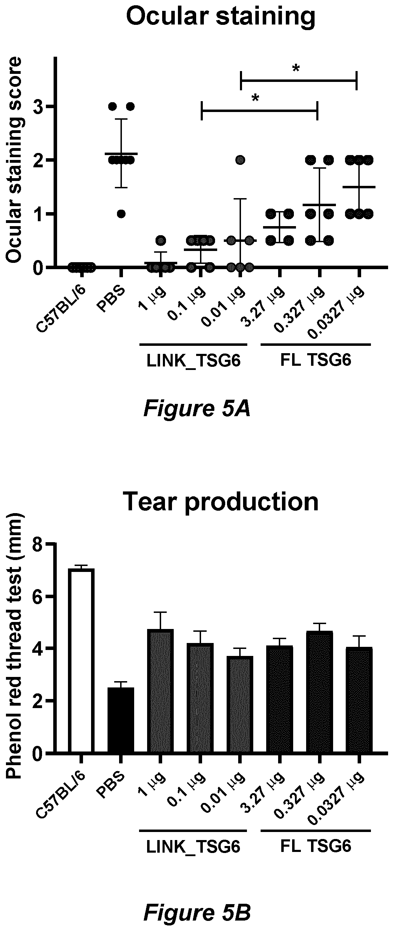

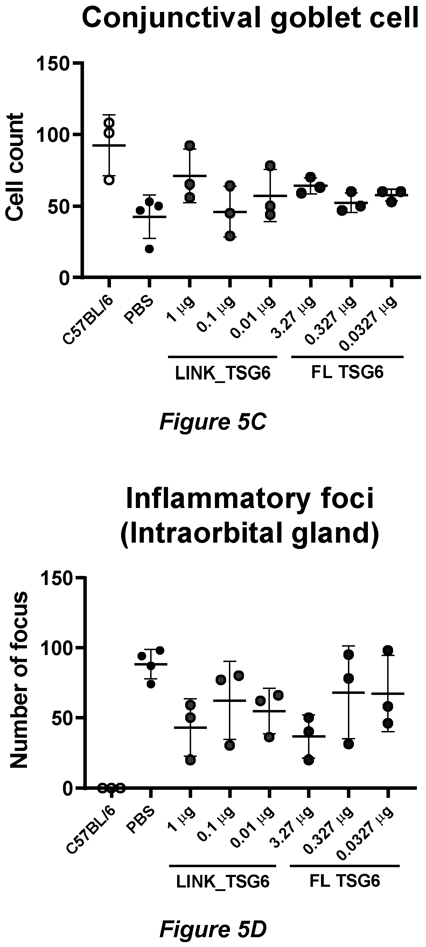

**Figure 5****.** LINK_TSG6 is more effective than full length human recombinant TSG-6 (FL TSG6) at reducing the signs of dry eye disease. A. Ocular staining score following lissamine green staining; B. Aqueous tear production as determined by phenol red thread test; C. Conjunctival goblet cell counts in PAS stained conjunctival sections D. Histological analysis of CD3 immunostaining in lacrimal gland as expressed by the number of foci with CD3 cell infiltration. Equivalent molar doses are compared (92, 9.2 and 0.92 pmol) corresponding to 1, 0.1 and 0.01 µg LINK_TSG6 and 3.27, 0.327 and 0.0327 µg FL TSG-6, respectively. Significance determined by one-way ANOVA and Tukey's multiple comparisons test. * = p<0.05.

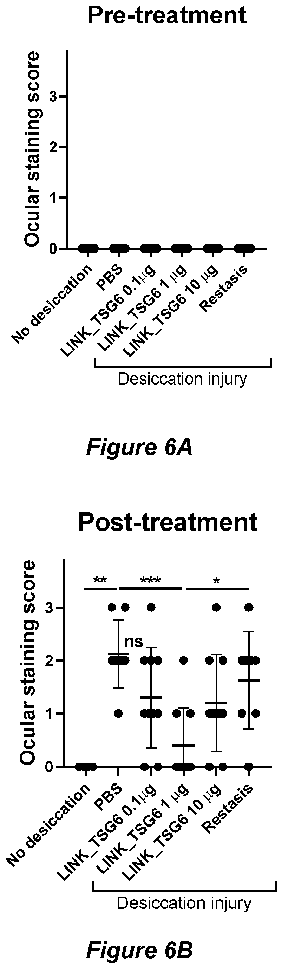

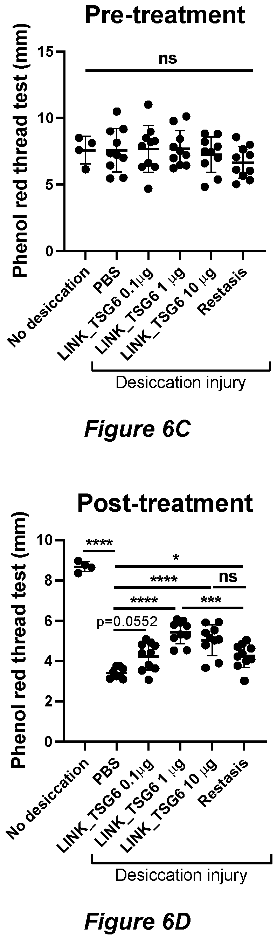

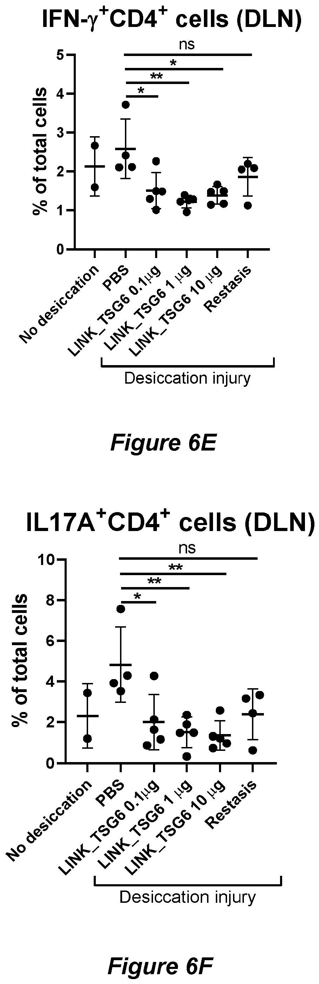

**Figure 6****.** Evaluation of LINK_TSG6 in a desiccation injury-induced dry eye model and comparison with Restasis. A. Ocular staining prior to the induction of desiccation injury and pre-treatment; B. Ocular staining post-desiccation injury and post-treatment; C. Tear production prior to the induction of desiccation injury and pre-treatment; D. Tear production post-desiccation injury and post-treatment; E. Th1 cells in draining cervical lymph nodes; F. Th17 cells in draining cervical lymph nodes. ns = not significant, p>0.05; * = p<0.05; ** = p<0.01 *** = p<0.001; **** = p<0.0001.

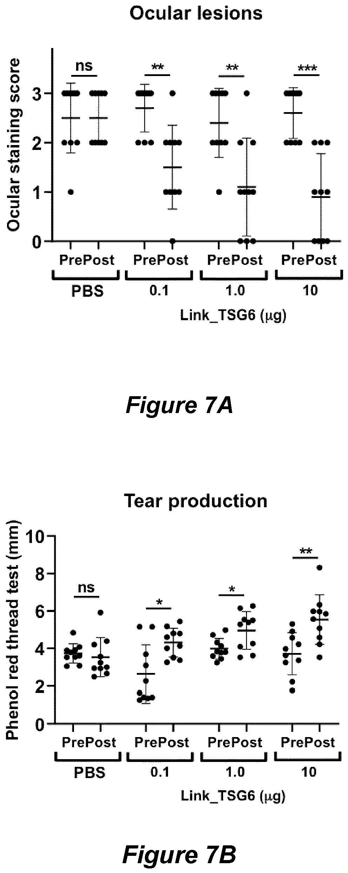

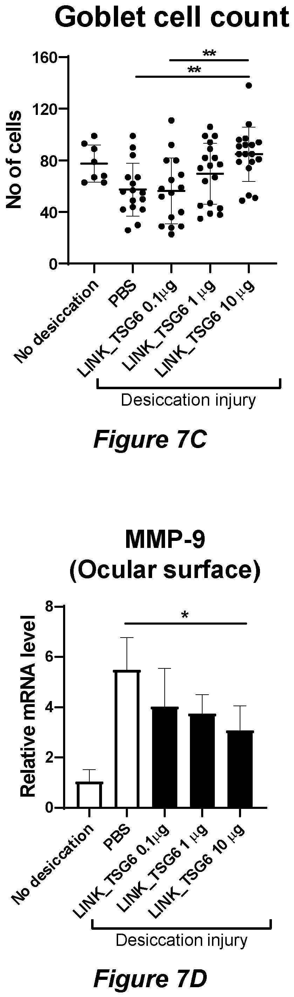

**Figure 7****.** Evaluation of LINK_TSG6 in already-desiccated mice. A. Ocular staining; B. Tear production; C. Conjunctival goblet cell counts in PAS stained conjunctival sections following treatment; D. MMP-9 mRNA levels at the ocular surface; ns = not significant, p>0.05; * = p<0.05; ** = p<0.01 *** = p<0.001; **** = p<0.0001. A&B Wilcoxon matched-pairs signed rank test for comparison between pre- and post-treatment. C&D one-way ANOVA and Tukey's multiple comparisons test.

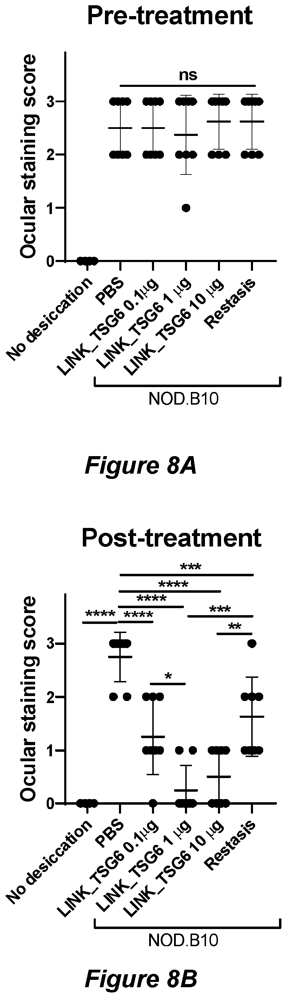

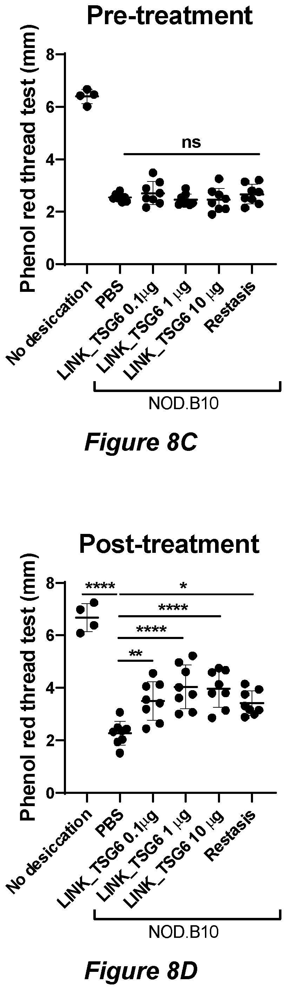

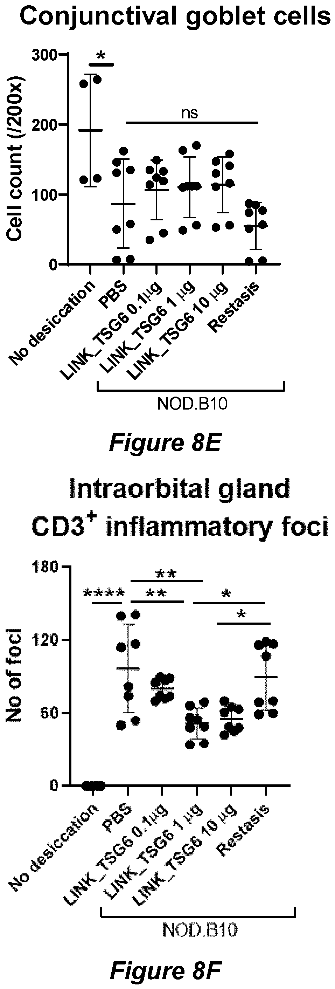

**Figure 8****.** Comparison of LINK_TSG6 and Restasis in a dry eye disease model. A. Ocular staining score following lissamine green staining, prior to treatment; B. Ocular staining score following lissamine green staining following treatment; C. Quantification of aqueous tear production by phenol red thread test pre-treatment D. Quantification of aqueous tear production by phenol red thread test post-treatment E. Conjunctival goblet cell counts in PAS stained conjunctival sections following treatment. F. Histological analysis of CD3 immunostaining in lacrimal gland. ns = not significant, p>0.05; * = p<0.05; ** = p<0.01 *** = p<0.001; **** = p<0.0001, one-way ANOVA and Tukey's multiple comparisons test.

### Detailed Description of the Invention

Aspects of the present disclosure will now be discussed with reference to the accompanying figures. Further aspects will be apparent to those skilled in the art.

The present disclosure provides a method for treating or preventing ocular surface disorders such as dry eye disease, which method comprises administering to a subject a LINK_TSG6 polypeptide. The inventors have shown that LINK_TSG6 is more potent than recombinant human TSG-6 in reducing corneal epithelial lesions and treating or reducing the signs or symptoms of ocular surface disorders such as dry eye disease. Without wishing to be bound by theory, this may be a result of improved penetration into the tissue (due to the smaller size of the molecule), differences in the biodistribution as compared to the full length protein, or the absence of signalling or enzymatic activities effected by the CUB_C domain. In this regard, full length TSG-6 binds more effectively to HA than LINK_TSG6 since the former interaction is cooperative (and likely involves CUB_C domain) (Baranova et al., 2011 J. Biol. Chem. 286, 25675-25686). HA has been implicated in some anti-inflammatory activities (see Day and Milner 2019).

### TSG-6 (Tumor Necrosis Factor-Stimulated Gene-6)

TSG-6 is a secreted protein composed of two modular domains. TSG-6 is not usually constitutively expressed in adult tissues, rather being induced in response to inflammatory mediators. During inflammation, TSG-6 is an endogenous protector of tissues. Many of the immunomodulatory and tissue-protective effects of MSCs are mediated by their secretion of TSG-6.

Recombinant full-length TSG-6 protein has been shown to have anti-inflammatory and tissue protective effects in a wide range of disease models, such as atherosclerosis, myocardial infarction, hypertrophic scarring, colitis, autoimmune diabetes, rheumatoid arthritis, traumatic brain injury or acute lung injury. Full length TSG-6 is hard to make, insoluble and prone to aggregation. As disclosed herein, these disadvantages are not associated with LINK_TSG6, a short recombinant peptide comprising the LINK module of human TSG-6. This short polypeptide is easier to make than full length TSG6, and is highly soluble and stable in solution.

LINK_TSG6 polypeptide as disclosed herein comprises only the Link module of human or mammalian TSG-6. In some aspects, the TSG-6 polypeptide comprises or consists essentially of the amino acid sequence according to SEQ ID NO: 2 or SEQ ID NO: 5. The Link module corresponds to residues 37-128 of SEQ ID NO:s 2 and 5, and is shown in SEQ ID NO: 7. In some preferred aspects, the LINK_TSG6 polypeptides useful in the present disclosure do not comprise some or all of residues 1-35 of the full length TSG6 sequence of SEQ ID NO: 2 or 5 at the N terminal,
The Link module is responsible for the hyaluronan (HA) binding activity, chondroitin-4-sulfate binding activity, aggrecan binding activity, inter-α-inhibitor (IαI) binding activity, bikunin binding activity, versican binding activity, dermatan sulfate binding activity, pentraxin-3 binding activity, thrombospondin-1 binding activity, thrombospondin-2 binding activity, fibronectin binding activity, heparin/heparan sulfate binding activity, RANKL binding activity of TSG-6, bone morphogenetic protein (BMP) -2 binding activity, BMP-4 binding activity, BMP-5 binding activity, BMP-6 binding activity, BMP-7 binding activity, BMP-13 binding activity, BMP-14 binding activity, CXCL4 binding activity, CXCL6 binding activity, CXCL8 binding activity, CXCL11 binding activity, CXCL12 binding activity, CCL2 binding activity, CCL5 binding activity, CCL7 binding activity, CCL19 binding activity, CCL21 binding activity or CCL27 binding activity.

LINK_TSG6 may be a fragment of TSG-6 exhibiting one or more of hyaluronan (HA) binding activity, chondroitin-4-sulfate binding activity, aggrecan binding activity, inter-α-inhibitor (IαI) binding activity, bikunin binding activity, versican binding activity, dermatan sulfate binding activity, pentraxin-3 binding activity, thrombospondin-1 binding activity, thrombospondin-2 binding activity, fibronectin binding activity, heparin/heparan sulfate binding activity, RANKL binding activity, bone morphogenetic protein (BMP) -2 binding activity, BMP-4 binding activity, BMP-5 binding activity, BMP-6 binding activity, BMP-7 binding activity, BMP-13 binding activity, BMP-14 binding activity, CXCL4 binding activity, CXCL6 binding activity, CXCL8 binding activity, CXCL11 binding activity, CXCL12 binding activity, CCL2 binding activity, CCL5 binding activity, CCL7 binding activity, CCL19 binding activity, CCL21 binding activity or CCL27 binding activity.

The LINK domain of TSG-6 (LINK_TSG6) may be the region of full-length TSG-6 N-terminal to the CUB_C domain. As such, the LINK_TSG6 protein may lack all or part of the CUB_C domain.

The LINK domain may contain the amino acid sequence of SEQ ID NO: 7 or SEQ ID NO: 9. LINK_TSG6 polypeptide comprises, consists, or consists essentially of (i) the amino acid sequence of SEQ ID NO: 7 or SEQ ID NO: 9, or (ii) an amino acid sequence having at least 80% identity to the amino acid sequence of SEQ ID NO: 7 or 9.

LINK_TSG6 is preferably a polypeptide comprising or consisting of: (i) the amino acid sequence of SEQ ID NO: 7 or 9, or (ii) an amino acid sequence having one of at least 80%, 85%, 90%, 91%, 92%, 93%, 94%, 95%, 96%, 97%, 98%, 99% or 100% identity to the amino acid sequence of SEQ ID NO: 7 or 9. Accordingly, the LINK_TSG6 polypeptide may comprise:
(a) the amino acid sequence of SEQ ID NO: 7;
(b) a variant thereof having at least 50% identity to the amino acid sequence of SEQ ID NO: 7 and having RANKL binding activity; or
(c) a fragment of either (a) or (b) having CXCL4 binding activity, CXCL6 binding activity, CXCL8 binding activity, CXCL11 binding activity, CXCL12 binding activity, CCL2 binding activity, CCL5 binding activity, CCL7 binding activity, CCL19 binding activity, CCL21 binding activity or CCL27 binding activity.. The LINK_TSG6 polypeptide may consists of, or consist essentially of, the sequence shown in SEQ ID NO: 7.

SEQ ID NO: 9 shows a recombinant polypeptide which includes the Link module of TSG-6 (LINK_TSG6). Accordingly, the TSG-6 polypeptide used in the disclosure may preferably comprises:
(a) the amino acid sequence of SEQ ID NO: 9;
(b) a variant thereof having at least 50% identity to the amino acid sequence of SEQ ID NO: 9 and having RANKL binding activity; or
(c) a fragment of either (a) or (b) having CXCL4 binding activity, CXCL6 binding activity, CXCL8 binding activity, CXCL11 binding activity, CXCL12 binding activity, CCL2 binding activity,CCL5 binding activity, CCL7 binding activity, CCL19 binding activity, CCL21 binding activity or CCL27 binding activity.

The LINK_TSG6 polypeptide preferably consists of, or consists essentially of, the sequence shown in SEQ ID NO: 9.

In some aspects described herein, the LINK_TSG6 polypeptide is not conjugated to an active agent, such as an antibody or antigen binding fragment. For example, in some cases, the LINK_TSG6 polypeptide is not conjugated to an IL-17A antibody or fragment thereof. In some cases, the LINK_TSG6 polypeptide comprises or consists of a single copy of LINK_TSG6 polypeptide, such as a single copy of SEQ ID NO: 7 or SEQ ID NO:9. In other words, in these cases, the LINK_TSG6 polypeptide does not comprise a plurality of LINK module sequences, such as a plurality of copies of SEQ ID NO: 7 or SEQ ID NO: 9. In some cases, the LINK_TSG6 polypeptide does not comprise a His tag, such as a 6xHIS tag.

Amino acid identity may be calculated using any suitable algorithm. For example the UWGCG Package provides the BESTFIT program which can be used to calculate homology (for example used on its default settings) (Devereux et al. (1984) Nucleic Acids Research 12, 387-395). The PILEUP and BLAST algorithms can be used to calculate homology or line up sequences (such as identifying equivalent or corresponding sequences (typically on their default settings), for example as described in Altschul (1993) J. Mol. Evol. 36, 290-300; Altschul et al. (1990) J. Mol. Biol. 215, 403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive- valued threshold score T when aligned with a word of the same length in a database sequence. T is referred to as the neighbourhood word score threshold (Altschul *et al.,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89, 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90, 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The variant sequences typically differ by at least 1, 2, 5, 10, 20, 30, 50 or more mutations (which can be substitutions, deletions or insertions of amino acids). For example, from 1 to 50, 2 to 30, 3 to 20 or 5 to 10 amino acid substitutions, deletions or insertions can be made. The modified polypeptide may generally retain CXCL4 binding activity, CXCL6 binding activity, CXCL8 binding activity, CXCL11 binding activity, CXCL12 binding activity, CCL2 binding activity, CCL5 binding activity, CCL7 binding activity, CCL19 binding activity, CCL21 binding activity or CCL27 binding activity, preferably in a dose-dependent manner. The substitutions are preferably conservative substitutions, for example according to the following Table. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

A LINK_TSG6 polypeptide used in the disclosure is typically at least 10, for example at least 15, 20, 25, 30, 40, 50, 60, 70, 80, 90 or more amino acids in length, up to 100, 150, 200 or 250 amino acids in length, as long as it retains the CXCL4 binding activity, CXCL6 binding activity, CXCL8 binding activity, CXCL11 binding activity, CXCL12 binding activity, CCL2 binding activity, CCL5 binding activity, CCL7 binding activity, CCL19 binding activity, CCL21 binding activity or CCL27 binding activity of TSG-6. Preferably, the polypeptide includes the sequence shown in SEQ ID NO: 7. Fragments of the amino acid sequence of SEQ ID NO: 2, SEQ ID NO: 3, SEQ ID NO: 5, SEQ ID NO: 6, SEQ ID NO: 7 or SEQ ID NO: 9 preferably contain the residues shown to be essential for hyaluronan binding in Mahoney et al. (2001) J. Biol. Chem. 276, 22764-22771 and Blundell et al. (2003) J. Biol. Chem. 278, 49261-49270. Fragments of the amino acid sequence of SEQ ID NO: 2 or 5 preferably contain the residues Lys-46 and/or Tyr-47 and/or Tyr-94 and/or Phe-105 and/or Tyr-113 of SEQ ID NO: 2 or 5. Most preferably, the fragment of SEQ ID NO: 2 or 5 contains each of residues Lys-46, Tyr- 47, Tyr-94, Phe-105 and Tyr-113 of SEQ ID NO: 2 or 5.

Fragments of the amino acid sequence of SEQ ID NO: 7 may be used in the disclosure. Such fragments preferably contain the residues Lys-10 and/or Tyr-11 and/or Tyr-58 and/or Phe-69 and/or Tyr-77 of SEQ ID NO: 7. Most preferably, the fragment of SEQ ID NO: 7 contains each of residues Lys-10, Tyr-11, Tyr-58, Phe-69 and Tyr-77 of SEQ ID NO: 7.

Fragments of the amino acid sequence of SEQ ID NO: 9 preferably contain the residues Lys-11 and/or Tyr-12 and/or Tyr-59 and/or Phe-70 and/or Tyr-78 of SEQ ID NO: 9. Most preferably, the fragment of SEQ ID NO: 9 contains each of residues Lys-11, Tyr-12, Tyr-59, Phe-70 and Tyr-78 of SEQ ID NO: 9. The TSG-6 polypeptides used in the disclosure may be chemically modified, e.g. post-translationally modified. For example, they may be glycosylated, phosphorylated or comprise modified amino acid residues. They may be modified by the addition of histidine residues to assist their purification or by the addition of a transmembrane sequence to promote insertion into the cell membrane. Such modified polypeptides fall within the scope of the term "polypeptide" used herein.

Suitable assays for determining the ability of a TSG-6 polypeptide to bind to HA, chondroitin-4-sulfate, aggrecan, inter-α-inhibitor (IαI), bikunin, versican, dermatan sulfate, pentraxin-3, thrombospondin-1, heparin/heparan sulfate, fibronectin and RANKL are well-known in the art (Getting et al. (2002) J. Biol. Chem. 277, 51068-51076; Mahoney et al. (2005) J. Biol. Chem. 280, 27044-27055; Salustri et al. (2004) Development 131, 1577-1586; Parkar et al. (1997) FEBS Lett. 410, 413-417; Parkar et al. (1998) FEBS Lett. 428, 171-176; Mahoney et al. (2001) J. Biol. Chem. 276, 22764-22771; Nentwich et al. (2002) J. Biol Chem. 211, 15354-15362; Kuznetsova et al. (2005) J. Biol. Chem. 280, 30899-30908), Dyer et al. (2014) J. Immunol 192, 2177-2185; Dyer et al. (2016) J. Biol. Chem. 291, 12627-12640; and Mahoney et al. (2008) J. Biol. Chem 283, 25952-25962.

TSG-6 polypeptides for use in the disclosure may be in a substantially isolated form. It will be understood that the polypeptide may be mixed with carriers or diluents which will not interfere with the intended purpose of the polypeptide and still be regarded as substantially isolated. A polypeptide for use in the disclosure may also be in a substantially purified form, in which case it will generally comprise the polypeptide in a preparation in which more than 50%, e.g. more than 80%, 90%, 95% or 99%, by weight of the polypeptide in the preparation is a polypeptide of the disclosure.

LINK_TSG6 polypeptides for use in the present disclosure may be natural or non-naturally occurring polypeptides. Polypeptides may be isolated from any suitable organism that expresses a TSG-6 polypeptide. The TSG-6 polypeptide may be isolated from a human or another suitable mammal, such as primates, rats or mice. Alternatively, TSG-6 polypeptide may be isolated from a fish or an amphibian. Polypeptides for use in the disclosure may also be prepared as fragments of such isolated polypeptides.

Further, the LINK_TSG6 polypeptides may also be made synthetically or by recombinant means. For example, a recombinant LINK_TSG6 polypeptide may be produced by transfecting cells in culture with an expression vector comprising a nucleotide sequence encoding the polypeptide operably linked to suitable control sequences, culturing the cells, extracting and purifying the LINK_TSG6 polypeptide produced by the cells. Methods for the recombinant production of polypeptides are well-known in the art (for example, Sambrook et al., 2001 , Molecular Cloning: a laboratory manual, 3rd edition, Cold Harbour Laboratory Press). Preferably, the LINK_TSG6 polypeptide is made in a bacteria, such as *E*. *coli.* Preferably, the LINK_TSG6 polypeptide is not made in a CHO cell or other mammalian cell. As will be appreciated by those skilled in the art, proteins and polypeptides made in a bacteria such as *E*. *coli* will completely or substantially lack glycosylation, whereas glycosylation is a common feature of proteins and polypeptides made in a mammalian cell such as a CHO cell. In particular, the LINK_TSG6 according to the present disclosure may lack N-linked glycosylation on Asn118 (SEQ ID NO: 2 or SEQ ID NO: 5).

The amino acid sequence of LINK_TSG6 polypeptides for use in the disclosure may be modified to include non-naturally occurring amino acids or to increase the stability of the compound. When the polypeptides are produced by synthetic means, such amino acids may be introduced during production. The polypeptides may also be modified following either synthetic or recombinant production. In some aspects, the LINK_TSG6 polypeptides described herein do not comprise a polyhistidine tag, such as a 6His tag. In some aspects, the LINK_TSG6 polypeptides described herein do not comprise a polyhistidine tag at the C-terminal of the polypeptide.

LINK_TSG6 polypeptides for use in the disclosure may also be produced using D- amino acids. In such cases the amino acids will be linked in reverse sequence in the C to N orientation. This is conventional in the art for producing such polypeptides.

A number of side chain modifications are known in the art and may be made to the side chains of the LINK_TSG6 polypeptides, provided that the polypeptides retain corneal defect healing activity.

### Ocular Surface Disorders

The present disclosure relates to the treatment or prevention of ocular surface disorders. Ocular surface disorders include Dry Eye Disease (DED), persistent corneal epitheliopathy (non-healing epithelial defects) associated with diabetic keratopathy, neurotrophic keratopathy, exposure keratopathy or limbal deficiency; contact lens-/eye drop-induced epithelial erosions; ocular graft versus host disease (GVHD); Stevens-Johnson syndrome (SJS); toxic epidermal necrolysis (TEN), ocular surface dysfunction in glaucoma patients, corneal wounds resulting from glaucoma surgery recurrent corneal erosion, superficial punctate keratitis, superior limbic keratoconjunctivitis.

### Dry Eye Disease

The present disclosure relates to the treatment or prevention of dry eye disease. Dry eye disease (also known as dry eye syndrome, or keratoconjunctivitis sicca) is the condition of having dry eyes, and affects 7-33% of the world population. Dry eye disease occurs when not enough tears are produced (aqueous deficient dry eye disease), or when tears evaporate too quickly (evaporative dry eye disease). Causes include aging (which is known to cause lacrimal grand atrophy and inflammation), infection, exposure to environmental irritants such as smoke, contact lens use, meibomian gland dysfunction, allergies, pregnancy, Sjögren's syndrome, vitamin A deficiency, laser eye treatment, or as a result of medication such as antihistamines, blood pressure medication, hormone replacement therapy and some antidepressants. In some case, dry eye disease occurs from activities that are associated with a reduced blinking rate, such as the use of screens such as computer monitors, smart phones or tablets, televisions or driving. Dry eye disease may result in tiny abrasions on the surface of the eyes (i.e. defects in corneal epithelium. Dry eye disease may result in pathologic changes in the corneal epithelium, such as squamous metaplasia and loss of goblet cells, in severe cases leading to corneal erosion, ulceration, neovascularization and scarring, or thinning and perforation. A diagnosis of dry eye disease may involve standardized dry eye questionnaire (The Ocular Surface Disease Index, or OSDI), a 12-item scale for the assessment of symptoms related to dry eye disease and their effect on vision.

Dry eye disease is a disease resulting from desiccation injury to the ocular surface, or inflammatory damage to the lacrimal gland. It is clinically distinct from other trauma induced ocular disorders such as chemical burn or trauma resulting from direct exposure of the ocular surface to chemicals (such as alcohol), physical or chemical debridement, blunt force trauma to the eye, penetrating eye injury, or other ocular wounding, although these trauma induced ocular conditions may result in the subsequent development of dry eye disease.

Signs and symptoms of dry eye disease include irritation, redness, discharge, easily fatigued eyes and blurred vision. The signs and symptoms range from mild and occasional to severe and continuous, and scarring of the cornea may result if left untreated.

As disclosed herein, the peptide LINK_TSG6 may be used to treat or prevent dry eye disease. Administration of LINK_TSG6 may result in a healing of corneal epithelial defects, an increase in tear production, the suppression of inflammation and/or an increase/preservation in the number of conjunctival goblet cells. The healing of corneal epithelial defects may result in a reduction in the number of corneal epithelial defects or the size of corneal epithelial defects. Preferably, the healing of corneal epithelial defects results in a reduction in the proportion of the corneal surface that comprises corneal epithelial defects.

Corneal epithelial defects are areas of epithelial (outermost corneal layer) loss, and may be due to mechanical trauma, corneal dryness, neurotrophic cornea, post surgical changes or any other of a variety of etiologies. LINK_TSG6 may be used to reduce or repair corneal epithelial defects. The presence or absence of corneal epithelial defects may be determined by scoring. Corneal epithelial defects may be visualised by staining. For example, through the use of lissamine green or fluorescein dye. The stain is applied to the cornea, and the area of epithelial defect is stained, thereby allowing visualisation of a defect.

An increase in tear production may be determined by Schirmer's test or phenol red thread test.

The number of goblet cell in the conjunctiva may be determined by impression cytology. A cellulose acetate filter paper is applied with pressure to the conjunctival surface for collection of superficial layer and subjected to PAS staining to stain mucin-secreting goblet cells. The number of goblet cells is calculated in the PAS-stained slide.

The presence, absence or amount of inflammation at the ocular surface may be determined by observation of conjunctival redness or conjunctival epithelial defects. Inflammation may cause disruption of corneal and conjunctival epithelium.

The level of inflammatory cytokines may be determined in a sample of tear from the patient. Tear samples may be obtained using Schirmer Tear Test strips. Nucleic acid and/or protein may be extracted from the tear strip by incubating the strip in ammonium bicarbonate and acetone. The level of inflammatory cytokines may be quantified or qualified by RT-PCR or ELISA. The inflammatory cytokines may be selected from IFN-y, TNF-α, IL-1β and IL-6. In some cases, the inflammatory cytokines may be quantified or qualified by immunoassay. In some cases, the level of MMP9 is determined in a semiquantitative manner (positive, trace, or negative) using an "InflammaDry" kit in human eyes.

The cornea is the transparent front part of the eye that covers the iris, pupil and the anterior chamber. The cornea, with the anterior chamber and lens, refracts light, with the cornea accounting for approximately two-thirds of the eye's total optical power. While the cornea contributes to most of the eye's focusing power, its focus is fixed. The cornea has unmyelinated nerve endings sensitive to touch, temperature and chemicals; a touch of the cornea causes an involuntary reflex to close the eyelid. Because transparency is of prime importance, the healthy cornea does not have or need blood vessels within it. Instead, oxygen dissolves in tears and then diffuses throughout the cornea to keep it healthy. Similarly, nutrients are transported via diffusion from the tear fluid through the outside surface and the aqueous humour through the inside surface. Nutrients also come via neurotrophins supplied by the nerves of the cornea. In humans, the cornea has a diameter of about 11.5 mm and a thickness of 0.5-0.6 mm in the center and 0.6-0.8 mm at the periphery. Transparency, avascularity, the presence of immature resident immune cells, and immunologic privilege makes the cornea a very special tissue.

The methods disclosed herein are particularly concerned with the corneal epithelium, and damage thereto. The corneal epithelium is an exceedingly thin (approximately 50 µm) multicellular epithelial tissue layer (non-keratinized stratified squamous epithelium) of fast-growing and easily regenerated cells, kept moist with tears. The corneal epithelium is made up of epithelial cells and covers the front of the cornea. It acts as a frontline barrier to protect the cornea, resisting the free flow of fluids from the tears, and prevents bacteria from entering the cornea and inside of the eye. Irregularity or defects of the corneal epithelium disrupts the smoothness of the air/tear-film interface, the most significant component of the total refractive power of the eye, thereby reducing visual acuity. It is continuous with the conjunctival epithelium, and is composed of about 6 layers of cells which are shed constantly on the exposed layer and are regenerated by multiplication in the basal layer. In dry eye disease, the corneal epithelium often becomes damaged.

In some methods, tear production is increased, or the thickness of the tear film coating the eye is increased. In some cases, tear retention is decreased, such that tears are evaporated quickly from the eye. Tearing (also known as lacrimation or lachrymation) is the reflex secretion of tears in response to external or internal irritants. Tears are a bodily fluid which may serve to clean and lubricate the eyes in response to irritation. In healthy mammalian eyes, the cornea is continually kept wet and nourished by basal tears. They lubricate the eye, and help to keep it clear of dust. Tear fluid contains water, mucin, lipids, lysozyme, lactoferrin, lipocalin, lacritin, immunoglobulins, glucose, urea, sodium, and potassium. Some of the substances in lacrimal fluid (such as lysozyme) fight against bacterial infection as a part of the immune system. Lysozyme does this by dissolving a layer in the outer coating, called peptidoglycan, of certain bacteria. Tears are a typical body fluid with a salt content similar to blood plasma. Usually, in a 24-hour period, 0.75 to 1.1 grams (0.03-0.04 ounce avoirdupois) of tears is secreted; this rate slows with age. In addition, the basal tears are composed of antioxidants such as ascorbate, urate, cysteine, glutathione, and tyrosine. Ascorbate and urate constitute half of the tears.

A second type of tears results from irritation of the eye by foreign particles, or from the presence of irritant substances such as onion vapors, perfumes and other fragrances, tear gas, or pepper spray in the eye's environment, including the cornea, conjunctiva, or nasal mucosa, which trigger TRP channels in the ophthalmic nerve. It can also occur with bright light and hot or peppery stimuli to the tongue and mouth. It is also linked with vomiting, coughing and yawning. These reflex tears attempt to wash out irritants that may have come into contact with the eye.

The methods disclosed herein may relate to preservation or improvement of tear production, such as basal tears or reflex tears.

### Patient selection

In accordance with the methods of the disclosure, the methods may additionally comprise the step of selecting a subject for treatment with a therapeutically effective amount of a polypeptide comprising or consisting of LINK_TSG6.

The methods may comprise evaluating a subject or patient for evidence of, or susceptibility to, an ocular surface disorder such as dry eye disease, such as corneal damage (for example the presence of corneal epithelial lesions), inadequate tear production and inflammation (for example redness or swelling, or the presence of one or more biological markers of inflammation such as one or more of the pro-inflammatory cytokines TNFα, IL-1β, IFN-γ or IL-6 and MMP9).

The method may involve a comprehensive eye examination. This may include: assessment of medical history to determine the patient's signs and symptoms and to note any general health problems, medications or environmental factors that may be contributing to the dry eye problem; external examination of the eye, including lid structure and blink dynamics; evaluation of the eyelids and cornea using bright light and magnification; or measurement of the quantity and quality of tears for any abnormalities.

An individual may be determined to have a corneal epithelial defect, for example through staining with lissamine green, rose bengal or fluorescein dye.

The individual may be determined to have inadequate tear production, for example through using the phenol red thread test or Schirmer's test.

In some cases described herein, the individual to be treated has a condition that is associated with increased incidence of ocular surface disorders such as dry eye disease. In some cases the condition is an autoimmune condition. The autoimmune condition may be Sjögren's syndrome. The individual may have been previously diagnosed as having Sjögren's syndrome. Sjögren's syndrome causes one of the most severe forms of dry eye disease, characterised by inflammatory destruction of lacrimal glands and the ocular surface. Inflammation of lacrimal glands and ocular surface is a key feature of dry eye disease, and plays an important role in the pathogenesis of dry eye disease and Sjögren's syndrome. In some cases, the autoimmune condition is rheumatoid arthritis or diabetes. In some cases, the individual has Type 1 diabetes or Type 2 diabetes.

In some cases, the ocular surface disorder such as dry eye disease does not involve inflammation. Dry eye disease not associated with inflammation may be associated with tear film instability and/or fast tear evaporation.

### Treatments

Disclosed herein are methods of treating or preventing ocular surface disorders such as dry eye disease. The methods may involve the reduction or elimination of one or more signs and symptoms of an ocular surface disorder such as dry eye disease, such as a reduction in corneal epithelial defects or lesions, an increase in tear production, an increase or preservation of the number of goblet cells, a reduction in the expression of one or more inflammatory cytokines, or a reduction in redness, or burning sensations, pain or discomfort, or an improvement in visual signs and symptoms such as loss of vision or blurring of vision.

The methods may involve the treatment or prevention of corneal epithelial defects or lesions. The methods may result in a decrease in the number and/or extent of corneal epithelial lesions. The methods may result in a decrease in the number and/or extent of corneal epithelial lesions as compared to the number and/or extent of corneal epithelial lesions prior to the treatment. The methods may result in a decrease in the number and/or extent of corneal epithelial lesions as compared to the number and/or extent of corneal epithelial lesions in an untreated control, or a control treated with full length TSG-6, such as TSG-6 comprising or consisting of SEQ ID NO: 3 or SEQ ID NO: 5. The extent of corneal epithelial lesions may be reduced by at least 25%, at least 50%, at least 75%, or at least 100% compared to the control. The treatment may result in corneal epithelial lesions on 0%, less than 10%, less than 20%, less than 30%, less than 40% or less than 50% of the corneal surface.

The methods may involve the treatment or prevention of inadequate tear production. The methods may result in an increase in tear production, such as an increase in the volume of tear production. The methods may result in an increase in the volume of tears produced as compared to the volume of tears prior to the treatment. The methods may result in an increase in the volume of tears produced as compared to the volume of tears in an untreated control, or a control treated with full-length TSG-6, such as TSG-6 comprising or consisting of SEQ ID NO: 3 or SEQ ID NO: 5. The volume of tears may be increased by at least 10%, at least 20%, at least 30%, at least 40%, at least 50%, at least 60%, at least 70%, at least 80%, at least 90%, at least 100% or more than 100% compared to the control.

The methods may involve increasing or preserving the number of goblet cells. In this context, increasing or preserving the number of goblet cells means that the number of goblet cells after treatment with LINK_TSG6 is not decreased to the same extent as the decrease observed in the absence of the treatment, such as in an untreated control. In some cases, the number of goblet cells is increased or unchanged as compared to the number of goblet cells prior to the treatment.

The methods may involve a reduction in the levels of one or more inflammatory cytokines. For example, the method may result in a reduction in the levels of nucleic acids corresponding to one or more inflammatory cytokines. In some cases, the method results in a reduction in the levels inflammatory cytokine proteins. The inflammatory cytokines may be selected from TNFα, IL6, IFN-y, and IL-1β. The levels may be decreased by 5%, 10%, 15%, 20%, 25%, 30%, 35%, 40%, 45%, 50%, 60%, 70%, 80%, 90% or 100% compared to the level before treatment, or as compared to an untreated control.

Treatment may result in complete resolution of the signs and symptoms of the ocular surface disorder such as dry eye disease.

Prevention may mean that no signs and symptoms of an ocular surface disorder such as dry eye disease emerge, or it may mean that the signs and symptoms of the ocular surface disorder such as dry eye disease develop to a lesser extent than in the absence of treatment.

Methods described herein may involve the topical administration of LINK_TSG6. LINK_TSG6 may be topically administered to the eye (ocular delivery), preferably to the cornea, such as the surface of the cornea. In some cases, the LINK_TSG6 is administered as an eye drop. The LINK_TSG6 may be formulated as a topical liquid formulation, such as an eye drop. LINK_TSG6 may be formulated by suspension or emulsion.

Administration is preferably in a therapeutically effective amount. A therapeutically effective amount of LINK_TSG6 may be determined according to various parameters, especially according to the polypeptide; the age, weight and condition of the patient to be treated; the route of administration; and the required regimen. Again, a physician will be able to determine the required route of administration and dosage for any particular patient. A therapeutically effective amount of LINK_TSG6 is an amount effective to ameliorate one or more signs and symptoms of an ocular surface disorder such as dry eye disease, such as to reduce the level of corneal damage or corneal lesions, increase lacrimal function, or increase lacrimal tear production, or reduce inflammation in the eye, such as inflammation in the cornea.

In some cases, LINK_TSG6 is administered at 12 µg-15 µg per eye, such as between 10 µg and 20 µg, or between 12 and 15 µg. In some cases, LINK_TSG6 is administered at about 8 µg per eye, about 9 µg per eye, about 10 µg per eye, about 11 µg per eye, about 12 µg per eye, about 13 µg per eye, about 14 µg per eye, or about 15 µg per eye.

In some cases, a higher dose is desired. In such cases, LINK_TSG6 is administered at about 120µg-150µg per eye. In some cases, the dosage of LINK_TSG6 is at least 80 µg, at least 90 µg, at least 100 µg, at least 110 µg, at least 120 µg, at least 130 µg, or at least 140 µg per eye. In some cases, the dosage of LINK_TSG6 is less than 170 µg, less than 160 µg, less than 150 µg, less than 140 µg, less than 130 µg or less than 120 µg per eye.

In some cases, an even higher dose is desired. For example, the dosage of LINK_TSG6 may be at least 170 µg, at least 180 µg, at least 190 µg, at least 200 µg, at least 210 µg at least 220 µg, at least 230 µg, at least 240 µg, at least 250 µg, at least 260 µg, at least 270 µg, at least 280 µg, at least 290 µg, at least 300 µg, at least 320 µg, at least 340 µg, at least 360 µg, at least 380 µg, least 400 µg, at least 420 µg, at least 440 µg, at least 460 µg, at least 480 µg, at least 500 µg, at least 550 µg, at least 600 µg, at least 650 µg, at least 700 µg, at least 750 µg, at least 800 µg, at least 900 µg, at least 1 mg, at least 1.1 mg, at least 1.2 mg, at least 1.3 mg, at least 1.4mg, at least 1.5mg, at least 1.6mg or at least 1.7mg.

Administration may be once daily, twice daily, three times daily, four times daily, five times daily, six times daily, seven times daily, eight times daily, nine times daily, ten times daily or more than ten times daily. Administration is preferably twice daily. In some preferred cases, the administration is more than twice daily, such as three or four times daily. In some preferred cases, the administration is less than four times daily, or less than three times daily, or two times daily or once daily. In particularly preferred cases, administration is two times daily. In some cases, the treatment comprises administering LINK_TSG6 polypeptide once per day. In some cases, the treatment comprises administering LINK_TSG6 polypeptide less frequently than once per day, such as once every two days, one time every three days, once every week, or once every two weeks.

The dose, schedule, mode or time course of administration of the polypeptide of the disclosure can be modified according to response to therapy. For example, dose and/or the frequency of administration and/or time course of administration may be increased if response to therapy is suboptimal. Conversely, dose and/or the frequency of administration and/or time course of administration can be reduced if response to therapy is better than expected.

In some cases, the treatment involves the co-administration of LINK_TSG6 with artificial tears, prednisolone, cyclosporine, Lifitegrast (Xiidra^{™}), or any combination thereof. Administration may be sequential or simultaneous. Preferably, where LINK_TSG6 is co-administered with one or more of prednisolone, cyclosporine, Lifitegrast (Xiidra^{™}) or artificial tears, the administration is simultaneous or substantially simultaneous. Preferably, the co-administered agents are administered via the same route of administration, such as by topical administration to the eye.

Evaluation of a subject for an ocular surface disorder such as dry eye disease may occur at any point before, during or after administration of a therapeutically effective amount of LINK_TSG6. In some aspects, the methods of the disclosure comprise commencing administration, evaluating a subject as above and based on the evaluation continuing, altering or discontinuing further administration. In some aspects, altering administration comprises increasing or decreasing the dose and/or the frequency and/or the time course of administration.

### Formulations

Formulations suitable for ocular administration include eye drops wherein the active compound is dissolved or suspended in a suitable carrier, especially an aqueous solvent for the active compound. In preferred formulations, LINK_TSG6 is solubilised in a saline solution. In some formulations LINK_TSG6 is solubilised in PBS (phosphate buffered saline). As described herein, the inventors have discovered that LINK_TSG6 polypeptide has high solubility in saline as compared to full length TSG6. Described herein are compositions, particularly pharmaceutical compositions, comprising high concentrations of LINK_TSG6.

The pharmaceutical composition may comprise LINK_TSG6 polypeptide solubilised in saline. The pharmaceutical composition may comprise LINK_TSG6 polypeptide solubilised in phosphate buffered saline. In some aspects, the pharmaceutical composition comprises at least 2000 µg/ml, 2100 µg/ml, 2200 µg/ml, 2300 µg/ml 2400µg/ml, 2500 µg/ml, 2600 µg/ml, 2700 µg/ml, 2800 µg/ml, 2900 µg/ml, 3000 µg/ml, 3100 pg/ml, 3200 µg/ml, 3300 µg/ml or more than 3300 µg/ml LINK_TSG6. Preferably, the pharmaceutical formation comprises at least 2000 µg/ml LINK_TSG6.

Eye drop formulations as disclosed herein may further comprise one or more of a preservative, antioxidant, stabilizer, tonicity modifier, viscosity modifier or buffer. Preferably, the eye drop formulation is a sterile eye drop formulation. In some cases, the eye drop formulation contains between about 240 µg/ml and about 300 µg/ml LINK_TSG6. Such formulations are useful for delivering between around 12 µg and around 15µg of LINK_TSG6 per drop. Each drop may be around 50 µl. The eye drop formulation may comprise between 200 µg/ml and 350 µg/ml, between 200 µg/ml and 320 µg/ml, between 220 µg/ml and 320 µg/ml, or between 240 µg/ml and 300 µg/ml. Preferably, the eye drop formulation comprises between about 240 µg/ml and about 300 µg/ml. The eye drop formulation may contain at least 200 µg/ml, at least 220 µg/ml, at least 230 µg/ml, at least 240 µg/ml, at least 250 µg/ml, at least 260 µg/ml, at least 270 µg/ml, at least 280 µg/ml, at least 290 µg/ml, at least 300 µg/ml, at least 310 µg/ml, at least 320 µg/ml or more than 320 µg/ml LINK_TSG6 polypeptide.

In some cases, a higher dose formulation is desired. Such formulations may contain between 2400 and 3000 µg/ml LINK_TSG6. Such formulations are useful for delivering between around 120 µg and around 150µg of LINK_TSG6 per drop. The eye drop formulation may comprise between 2000 µg/ml and 3500 µg/ml, between 2000 µg/ml and 3200 µg/ml, between 2200 µg/ml and 3200 µg/ml, or between 2400 µg/ml and 3000 µg/ml. Preferably, the eye drop formulation comprises between about 2400 µg/ml and about 3000 µg/ml. The eye drop formulation may contain at least 2000 µg/ml, at least 2200 µg/ml, at least 2300 µg/ml, at least 2400 µg/ml, at least 2500 µg/ml, at least 2600 µg/ml, at least 2700 µg/ml, at least 2800 µg/ml, at least 2900 µg/ml, at least 3000 µg/ml, at least 3100 µg/ml, at least 3200 µg/ml or more than 3200 µg/ml LINK_TSG6 polypeptide.

In the some cases, the eye drop formulation may further comprise artificial tears. Artificial tears are lubricant eye drops. Artificial tears may contain one or more agents selected from carboxymethyl cellulose, polyvinyl alcohol, hydroxypropyl methylcellulose, hydroxypropyl cellulose, hyaluronan, water, salts and polymers, such as polyethylene glycol or polypropylene glycol.

In some cases, the eye drop formulation may further comprise prednisolone, cyclosporine, Lifitegrast (Xiidra^{™}) or a combination of prednisolone and cyclosporine, prednisolone and Lifitegrast (Xiidra^{™}), cyclosporine and Lifitegrast (Xiidra^{™}), or prednisolone, cyclosporine and Lifitegrast (Xiidra^{™}).

Pharmaceutical compositions may be prepared using a pharmaceutically acceptable "carrier" composed of materials that are considered safe and effective. "Pharmaceutically acceptable" refers to molecular entities and compositions that are "generally regarded as safe", e.g., that are physiologically tolerable and do not typically produce an allergic or similar untoward reaction, such as gastric upset, loss, or change, of taste (ageusia) and the like, when administered to a human. In some aspects, this term refers to molecular entities and compositions approved by a regulatory agency of the US federal or a state government, as the GRAS list under section 204(s) and 409 of the Federal Food, Drug and Cosmetic Act, that is subject to premarket review and approval by the FDA or similar lists, the U.S. Pharmacopeia or another generally recognised pharmacopeia for use in animals, and more particularly in humans.

The term "carrier" refers to diluents, binders, lubricants and disintegrants. Those with skill in the art are familiar with such pharmaceutical carriers and methods of compounding pharmaceutical compositions using such carriers.

The pharmaceutical compositions provided herein may include one or more excipients, e.g., solvents, solubility enhancers, suspending agents, buffering agents, isotonicity agents, antioxidants or antimicrobial preservatives. When used, the excipients of the compositions will not adversely affect the stability, bioavailability, safety, and/or efficacy of the active ingredients, i.e. LINK_TSG6 used in the composition. Thus, the skilled person will appreciate that compositions are provided wherein there is no incompatibility between any of the components of the dosage form. Excipients may be selected from the group consisting of buffering agents, solubilizing agents, tonicity agents, chelating agents, antioxidants, antimicrobial agents, and preservatives. Ointments are typically prepared from the active compound and a paraffinic or a water-miscible ointment base.

Creams are typically prepared from the active compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

Emulsions are typically prepared from the active compound and an oily phase, which may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary aspects described above, many equivalent modifications and variations will be apparent to those skilled in the art when given this disclosure. Accordingly, the exemplary aspects of the disclosure set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by the use of the antecedent "about," it will be understood that the particular value forms another aspect. The term "about" in relation to a numerical value is optional and means for example +/- 10%.

### Examples

### EXAMPLE 1: LINK TSG6 is more soluble than full length TSG-6

The solubility and aggregation state of full-length TSG-6 and LINK_TSG6 in PBS were compared using UV spectrophotometry and dynamic light scattering (DLS) at a range of concentrations. The LINK_TSG6 was fully soluble at 2 mg/ml and showed no aggregation at 0.4, 0.8, 1.6 or 3.2 mg/ml. The full-length protein had much lower solubility with less than 40% of the protein remaining in solution at 0.4, 0.8, 1.6. and 3.2 mg/ml. The full-length protein was also highly aggregated at 0.2, 0.4. 0.8, 1.6 and 3.2 mg/ml. The DLS measurements were out of range for full-length TSG-6 at 1.6 and 3.2 mg/ml, indicating that aggregates of greater than 25 million Da were being formed.

### EXAMPLE2: LINK TSG6 improves signs and symptoms of dry eye disease in a mouse model

Topical TSG-6 has previously been shown to be as effective in inflammation mediated dry eye disease as cyclosporine eye drops (Kim *et al.,* 2016). To test whether the LINK_TSG6 polypeptide was useful in this indication we investigated its effects in the NOD.B10 mouse model of primary ocular Sjögren's syndrome (spontaneous dry eye disease without diabetes).

NOD.B10.H2^{b} mice (12 week-old, Jackson Lab), a model for primary ocular Sjögren's syndrome (dry eye disease without diabetes) were treated with topically administered with LINK_TSG6 for 7 days. C57BL/6 mice were used as a negative control, as they do not develop spontaneous dry eye Mice were treated by topical application of 1 µg LINK_TSG6, in 10 µl PBS, 4x per day (QID) for 7 days. Mice were anesthetised with an intraperitoneal injection of zolazepam-tiletamine (Zoletil^{®}, Virbac, Carros, France), and either 10 µl LINK_TSG6 or PBS was administered using a pipette. Mice were randomly assigned to treatment groups as follows:
1) Group 1 (Negative control): C57BL/6 mice, 12-week old (n=6, 12 eyes), untreated
2) Group 2 (Positive control): NOD.B10, 12-week old (n=6, 6 eyes) + PBS 10 µl QID
3) Group 3 (Experimental group): NOD.B10, 12-week old (n=8, 8 eyes) + LINK_TSG6 1 µg (in 10 µl PBS) QID.

To assess the effect of treatment on dry eye disease, we undertook the following assays:
1) Corneal epithelial defects by vital staining (with lissamine green) of defect sites and scoring. After applying one drop of 3% lissamine Green B to the inferior lateral conjunctival sac of a mouse, the dye staining of the corneal surface was graded in a blinded manner by two ophthalmologists as per the following ocular staining score system: score 0 for no punctuate staining; score 1 when less than one third of the corneal surface was stained; score 2 when two thirds or less was stained; and score 3 when more than two thirds was stained;
2) Lacrimal tear production examined by phenol red thread test;
3) Inflammatory cytokine expression by real time RT PCR in the cornea and intra-orbital gland; and
4) Conjunctival goblet cell count on PAS-stained conjunctival fornix. The number of PAS-stained cells was counted per 100 µm in four different sections of the eye from the same animal, and the average count was determined in each eye as the goblet cell density.

As shown in Figure 2A, after LINK_TSG6 treatment, corneal epithelial defects were significantly reduced (p < 0.01). There was a significant improvement in corneal epithelial defects between PBS- and Link_TSG6- treated groups (p < 0.001).

As shown in Figure 2B, tear production was significantly increased by LINK_TSG6 treatment (p < 0.01). There was a significant improvement in tear production between PBS- and LINK_TSG6-treated groups (p < 0.001).

Figure 2C shows that LINK_TSG6 treatment significantly suppressed mRNA levels of IFN-γ, TNF-α and IL-1β in the cornea and intraorbital gland. LINK_TSG6-treated eyes had levels of cytokines similar to those of control C57BL/6 mice.

2D shows that LINK_TSG6 treatment significantly increased the number of conjunctival goblet cells (these cells produce the mucin components in tears).

Overall, these results demonstrate that LINK_TSG6 significantly suppressed inflammation, reduced corneal epithelial defects, increased tear production, and increased conjunctival goblet cells in NOD.B10 dry eye mice.

### EXAMPLE 3: Effects of LINK TSG6 on dry eye disease are dose-dependent

We were interested to understand whether the effects observed in example 1 were dose dependent. To this end, we applied 1, 0.1, or 0.01 µg of LINK_TSG6, in 5 µl PBS, topically QID for 7 days to 12 week old NOD.B10.H2b mice (Jackson Lab).

Mice were randomly assigned to the following treatment groups:
1) Group 1 (Negative control): C57BL/6 mice, 12-week old (n=6, 12 eyes) untreated
2) Group 2 (Positive control): NOD.B10 (n=8, 8 eyes) + PBS 5 µl QID
3) Group 3 (Experimental group): NOD.B10 (n=8, 8 eyes) + LINK_TSG6 1 µg (in 5 µl PBS) QID
4) Group 4 (Experimental group): NOD.B10 (n=8, 8 eyes) + LINK_TSG6 0.1 µg QID
5) Group 5 (Experimental group): NOD.B10 (n=8, 8 eyes) + LINK_TSG6 0.01 µg QID

The effect of the treatment was determined as per example 1.

As shown in Figure 3A, corneal epithelial defects were quantified in lissamine green-stained corneas. LINK_TSG6 1 µg and 0.1 µg was effective in reducing corneal epithelial defects, but LINK_TSG6 0.01 µg did not significantly improve corneal epithelial defects.

In Figure 3B, Phenol red threat test indicated that LINK_TSG6 1 µg and 0.1 µg was were effective in increasing the amount of tear production, but LINK_TSG6 0.01 µg did not significantly improve the amount of tear production.

As shown in Figures 3C and D, the highest dose of LINK_TSG6 tested, 1 µg was most effective in suppressing TNF-α expression.

Overall, these results demonstrate a dose-dependent improvement of dry eye parameters by topically applied LINK_TSG6 polypeptide (1 µg most effective and 0.01 µg least effective).

These effects were also observed with higher doses of LINK_TSG6. As shown in Figure 4A, the higher doses (1 µg and 10 µg) of LINK_TSG6 exhibited significantly less corneal epithelial damage as compared to PBS-treated mice. At the highest dose, many of the LINK_TSG6 treated eyes have no corneal lesions (score of 0), which is the same as the untreated negative control C57BL/6 mice. This shows that LINK_TSG6 can promote the healing or repair of epithelial lesions.

Dose dependent responses were also observed in respect of tear production (Figure 4B - note that even the lowest dose of LINK_TSG6 tested has a significant effect on tear production), inflammation (Figure 4C) and conjunctival goblet cell numbers (Figure 4D).

### EXAMPLE 4: Comparison of effects of full length TSG-6 protein and LINK TSG6 on dry eye disease

Our experiments revealed that LINK_TSG6 polypeptide was effective at treating dry eye disease. We were interested to understand how this compared to full length (FL) TSG-6 (FL TSG6). We compared equivalent molar doses of FL TSG6 and LINK_TSG6, administered 2x/day (BID).

12 week old NOD.B10 mice and C57BL/6 mice were randomly assigned to treatment groups as follows:
1) Group 1 NOD.B10 + PBS 5 µl BID for 7 days (positive control);
2) Group 2 NOD.B10 + full-length TSG-6 (FL TSG6) (R&D Systems) 5 µl (3.27, 0.327, 0.0327µg) BID for 7 days;
3) Group 3 NOD.B10 + LINK_TSG6 5 µl (1, 0.1, 0.01 µg) BID for 7 days; and
4) Group 4 C57BL/6 mice (negative control, no dry eye).

Dosages of 0.01, 0.1 and 1.0 micrograms LINK_TSG6 were chosen as per example 2. Mass spectrometry was used to determine the molecular weight of recombinant human TSG-6 (R&D Systems) as 35.7 kDa. Based on this determination, we calculated equimolar doses of 0.037, 0.37 and 3.7 micrograms for full-length TSG-6 (FL TSG6).

Signs and symptoms of dry eye disease were determined as per the previous examples, namely:
1) Lissamine green staining and score for corneal epithelial damage. After applying one drop of 3% Lissamine Green B to the inferior lateral conjunctival sac of a mouse, the dye staining of the corneal surface was graded in a blinded manner by two ophthalmologists as per the following ocular staining score system: score 0 for no punctuate staining; score 0.5 for trace staining; score 1 when less than one third of the corneal surface was stained; score 2 when two thirds or less was stained; and score 3 when more than two thirds was stained;
2) Phenol red thread test for lacrimal tear secretion
3) Histology (conjunctival PAS staining, lacrimal gland CD3 immunostaining)

As shown in Figure 5A, LINK_TSG6 resulted in a significant reduction in corneal epithelial lesions as compared to an equimolar concentration of full length TSG-6.

Figures 5B, 5C and 5D show that the highest dose tested, 1 µg LINK_TSG6, caused increased tear production, preserved goblet cells and reduced the number of CD3-stained inflammatory foci in the intraorbital gland, as compared to an equimolar dose (3.27 µg) of full length TSG-6.

### EXAMPLE 5: Evaluation of LINK TSG6 in a desiccation injury-induced dry eye model and comparison with Restasis.

Having demonstrated the efficacy of LINK_TSG6 in the NOD.B10 mouse model of primary ocular Sjögren's syndrome (spontaneous dry eye disease without diabetes), we were interested to understand whether LINK_TSG6 was also effective in treating individuals with the prevalent environmental evaporative dry eye disorder.

We use a desiccation model that better emulates an evaporative dry eye which is more prevalent form of dry eye, compared to the less prevalent Sjogren's syndrome-like dry eye. 7 week-old C57BL/6 mice were kept in a dry chamber and injected intraperitoneally with scopolamine three times daily for 10 days to induce desiccation injury. The air flow from an electric fan was allowed into the cage through the screen for 24 h, and humidity was maintained 30-35% inside the cage. Mice were divided into treatment groups as follows:
1) No desiccation injury (negative control, 2 mice, 4 eyes)
2) Group 1 (5 mice, 10 eyes): Desiccation injury + PBS 5 µl BID for 10 days (positive control)
3) Group 2 (5 mice, 10 eyes): Desiccation injury + LINK_TSG6 0.1 µg (in PBS 5 µl) BID for 10 days
4) Group 3 (5 mice, 10 eyes): Desiccation injury + LINK_TSG6 1 µg (in PBS 5 µl) BID for 10 days
5) Group 4 (5 mice, 10 eyes): Desiccation injury + LINK_TSG6 10 µg (in PBS 5 µl) BID for 10 days
6) Group 5 (5 mice, 10 eyes): Desiccation injury + Restasis (0.05% cyclosporine A) 5 µL BID for 10 days

After 10 days, signs and symptoms of dry eye were evaluated as follows;
1) Fluorescein staining and score for corneal epithelial damage (C57BL/6 mice are black and fluorescein stain is better for visualization than lissamine green)
2) Phenol red thread test for lacrimal tear secretion
3) Histology (conjunctival PAS staining, lacrimal gland CD3 immunostaining)
4) Molecular assay of ocular surface and lacrimal glands (real time RT-PCR for inflammatory cytokines)

Desiccation injury significantly induced corneal epithelial defects in C57BL/6 mice (p<0.01), as shown in Figure 6A (pre-desiccation and pre-treatment, showing animals selected for treatment had no corneal lesions at the start of the study) and 6B (post-desiccation and post-treatment), and LINK_TSG6 1 µg was effective at decreasing corneal epithelial defects under desiccation (p<0.001). Interestingly, Restasis did not significantly reduce corneal epithelial defects in this model, and LINK_TSG6 1 µg was better at decreasing corneal epithelial defects than Restasis (p<0.05).

As shown in Figure 6C (pre-desiccation and pre-treatment) and 6D (post-desiccation and post-treatment), desiccation injury significantly reduced tear production in C57BL/6 mice (p<0.0001). As with the mouse model of primary ocular Sjögren's syndrome, LINK_TSG6 1 µg and 10 µg were effective at preserving tear production under desiccation (p<0.0001), and even 0.1 µg of LINK_TSG6 preserved tear production (p=0.0552). Although Restasis was effective at preserving tear production under desiccation (p<0.05), interestingly LINK_TSG6 1 µg was better at preserving tear production than Restasis (p<0.001).

It is well-known that Th1 and Th17 cells are increased in draining cervical lymph nodes (DLN) under desiccation stress, and that these cells are responsible for inducing dry eye. We therefore performed FACS analysis on cervical draining lymph nodes to determine the level of Th1 (IFN-γ⁺CD4⁺ cells) and Th17 cells (IL17A⁺CD4⁺ cells) in response to the various treatments. As shown in Figures 6E and 6F, LINK_TSG6 was also effective at suppressing Th1 and Th17 cells in cervical lymph nodes. Restasis had no such suppressive effect.

These data therefore indicate that LINK_TSG6 is also effective at treating dry eye disease, including in individuals with the prevalent environmental evaporative dry eye.

### EXAMPLE 6: Evaluation of LINK TSG6 in established desiccation injury-induced dry eye

Having demonstrated that LINK_TSG6 is effective at treating dry eye disease, including in individuals with the prevalent environmental evaporative dry eye, we were interested to understand whether LINK_TSG6 was also effective in treating individuals with established dry eye, to reverse the effects of the disease.

We used the same desiccation model as in Example 5, this time allowing the dry eye disease to become established for one week prior to treatment with LINK_TSG6. LINK_TSG6 was applied to the ocular surface after desiccation injury had been exerted to the ocular surface. The mice were then kept in the dry chamber with scopolamine injections for the 10 further days during which the treatments were administered.

7 week-old C57BL/6 mice were kept in a dry chamber and injected intraperitoneally with scopolamine three times daily for 7 days to establish dry eye. Mice were divided into treatment groups as follows:
1) No desiccation injury (negative control, 2 mice, 4 eyes)
2) Group 1 (5 mice, 10 eyes): Desiccation injury + PBS 5 µl BID for 10 days (positive control)
3) Group 2 (5 mice, 10 eyes): Desiccation injury + LINK_TSG6 0.1 µg (in PBS 5 µl) BID for 10 days
4) Group 3 (5 mice, 10 eyes): Desiccation injury + LINK_TSG6 1 µg (in PBS 5 µl) BID for 10 days
5) Group 4 (5 mice, 10 eyes): Desiccation injury + LINK_TSG6 10 µg (in PBS 5 µl) BID for 10 days.

Signs and symptoms of dry eye disease were determined as per the previous examples, namely:
1) Fluorescein staining and score for corneal epithelial damage (B6 mice are black and fluorescein stain is better for visualization than lissamine green);
2) Phenol red thread test for lacrimal tear secretion; and
3) Histology (conjunctival PAS staining, lacrimal gland CD3 immunostaining).

As shown in Figure 7A LINK_TSG6 was effective at decreasing corneal epithelial defects at all concentrations tested. As shown in Figure 7B, at all concentrations tested, LINK_TSG6 was effective at increasing aqueous tear production. Significant changes were observed even for the lowest concentration tested.

Figure 7C demonstrates that 1 µg and 10 µg of LINK_TSG6 resulted in an increase in goblet cell number as compared to control, restoring to levels comparable to those observed in the absence of desiccation.

Moreover, as shown in Figure 7D, treatment with LINK_TSG6 was effective at suppressing the level of MMP-9 mRNA in the ocular surface.

These data therefore indicate that LINK_TSG6 is effective at treating established dry eye disease, in addition to reducing the severity of desiccation injury. These data support the use of LINK_TSG6 for the treatment of individuals with dry eye disorder, including in individuals with the prevalent environmental evaporative dry eye.

### EXAMPLE 7: Evaluation of LINK TSG6 in a dry eye model and comparison with Restasis.

Having demonstrated the superiority of LINK_TSG6 to Restasis in treating evaporative dry eye, we were interested to compare LINK_TSG6 to Restasis in the NOD.B10 mouse model of primary ocular Sjögren's syndrome (spontaneous dry eye disease without diabetes), as used in Example 4.

12 week old NOD.B10 mice and C57BL/6 mice were randomly assigned to treatment groups as follows:
1) Group 1 C57BL/6 mice (negative control, no dry eye/desiccation).
2) Group 2 NOD.B10 + PBS 5 µl BID for 7 days (positive control);
3) Group 3 NOD.B10 + LINK_TSG6 5 µl (0.1, 1, 10 µg/5 µl) BID for 7 days; and
4) Group 4 NOD.B10 + Restasis 5 µl BID for 7 days;

Signs and symptoms of dry eye disease were determined as per the previous examples, namely:
1) Lissamine green staining and score for corneal epithelial damage.
2) Phenol red thread test for lacrimal tear secretion
3) Histology (conjunctival PAS staining, lacrimal gland CD3 immunostaining)

As shown in Figure 8B and D, and in agreement with our studies in Example 4, LINK_TSG6 resulted in a significant reduction in corneal epithelial lesions and increase in tear production in the NOD.B10 mouse model of primary ocular Sjogren's syndrome. Moreover, LINK_TSG6 promoted healing to a greater extent than Restasis at both the 1 and 10 µg doses, resulting in reduced corneal lesions, preservation in the number of goblet cells, and reduced number of CD3-stained inflammatory foci in the intraorbital gland, as compared to Restasis. This shows that even at the lowest doses tested, LINK_TSG6 was better at treating dry eye in the NOD.B10 mouse model of primary ocular Sjögren's syndrome (spontaneous dry eye disease without diabetes) than Restasis, as previously observed in preventing the development of, or treating established, evaporative dry eye disease. These data further support the use of LINK_TSG6 for the treatment of individuals with dry eye disorder.

## Claims

1. LINK_TSG6 polypeptide for use in the treatment or prevention of an ocular surface disorder such as dry eye disease, wherein the LINK_TSG6 polypeptide consists of (i) the amino acid sequence of SEQ ID NO:7 or SEQ ID NO:9, or (ii) an amino acid sequence up to 100 amino acids in length having at least 90% identity to the amino acid sequence of SEQ ID NO:7 or SEQ ID NO:9.

2. The LINK_TSG6 polypeptide for use according to claim 1, wherein the treatment or prevention of an ocular surface disorder such as dry eye disease comprises one or more effects selected from the group consisting:
healing of corneal epithelial defects;
increase in tear production;
suppression of inflammation; and
a higher number of conjunctival goblet cells.

3. The LINK_TSG6 polypeptide for use according to claim 1 or claim 2 wherein the treatment comprises topical administration of LINK_TSG6 polypeptide to the eye.

4. The LINK_TSG6 polypeptide for use according to any one of claims 1 to 3 wherein the treatment comprises administering LINK_TSG6 polypeptide two times per day, optionally wherein the treatment comprises administering LINK_TSG6 polypeptide more than two times per day, optionally wherein the treatment comprises administering LINK_TSG6 polypeptide less than four times per day.

5. The LINK_TSG6 polypeptide for use according to claim 3 wherein the LINK_TSG6 polypeptide is formulated as an eye drop.

6. The LINK_TSG6 polypeptide for use according to any one of the preceding claims wherein LINK_TSG6 is co-administered with one or more of prednisolone, cyclosporine, Lifitegrast (Xiidra^{™}) or artificial tears.

7. The LINK_TSG6 polypeptide for use according to any one of the preceding claims wherein about 10µg-200µg LINK_TSG6 polypeptide is administered per eye, preferably 120µg-150µg LINK_TSG6 polypeptide is administered per eye.

8. The LINK_TSG6 polypeptide for use according to claim 2, wherein the suppression of inflammation comprise a decrease in the production of one or more pro-inflammatory cytokines in the cornea or the intraorbital lacrimal glands, the pro-inflammatory cytokines optionally selected from TNF-α, IL-6, IFN-γ and IL-1β.

9. The LINK_TSG6 polypeptide for use according to claim 2 wherein the treatment or prevention of dry eye disease comprises healing of corneal epithelial defects, increase in tear production, suppression of inflammation or increase in number of conjunctival goblet cells as compared to the corneal epithelial defects, tear production, inflammation or number of conjunctival goblet cells prior to the administration of LINK_TSG6 polypeptide.

10. The LINK_TSG6 polypeptide for use according to claim 2 wherein the treatment or prevention of dry eye disease comprises healing of corneal epithelial defects, increase in tear production, suppression of inflammation or increase in number of conjunctival goblet cells as compared to the corneal epithelial defects, tear production, inflammation or number of conjunctival goblet cells of a control individual treated with full-length TSG-6 protein.

11. The LINK_TSG6 polypeptide for use according to any one of the preceding claims wherein the individual being treated has Sjögren's syndrome.

12. The LINK_TSG6 polypeptide for use according to any one of the preceding claims wherein the treatment comprises the administration of an eye drop comprising LINK_TSG6.

13. An eye drop formulation comprising LINK_TSG6 polypeptide, wherein the LINK_TSG6 polypeptide consists of (i) the amino acid sequence of SEQ ID NO:7 or SEQ ID NO:9, or (ii) an amino acid sequence up to 100 amino acids in length having at least 90% identity to the amino acid sequence of SEQ ID NO:7 or SEQ ID NO:9.

14. The eye drop formulation according to claim 13 comprising between 240µg/ml and 3000µg/ml, preferably between 2400µg/ml and 3000µg/ml LINK_TSG6 polypeptide.

15. The eye drop formulation according to claim 13 or claim 14 further comprising prednisolone, cyclosporine, Lifitegrast or artificial tears.

## Patentansprüche

1. LINK_TSG6-Polypeptid zur Verwendung bei der Behandlung oder Prävention einer okularen Oberflächenstörung wie des Syndroms des trockenen Auges, wobei das LINK_TSG6-Polypeptid aus (i) der Aminosäuresequenz der SEQ ID NO: 7 oder SEQ ID NO: 9 oder (ii) einer Aminosäure mit einer Länge von bis zu 100 Aminosäuren und zumindest 90 % Identität mit der Aminosäuresequenz der SEQ ID NO: 7 oder SEQ ID NO: 9 besteht.

2. LINK_TSG6-Polypeptid zur Verwendung nach Anspruch 1, wobei die Behandlung oder Prävention einer okularen Oberflächenstörung wie des Syndroms des trockenen Auges eine oder mehrere aus folgender Gruppe ausgewählte Wirkungen umfasst:
Heilung von Hornhautepitheldefekten;
Steigerung der Tränenproduktion;
Unterdrückung einer Entzündung; und
eine höhere Anzahl von Becherzellen in der Bindehaut.

3. LINK_TSG6-Polypeptid zur Verwendung nach Anspruch 1 oder Anspruch 2, wobei die Behandlung eine topische Verabreichung eines LINK_TSG6-Polypeptids an das Auge umfasst.

4. LINK_TSG6-Polypeptid zur Verwendung nach einem der Ansprüche 1 bis 3, wobei die Behandlung eine Verabreichung eines LINK_TSG6-Polypeptids zweimal täglich umfasst, wobei die Behandlung gegebenenfalls die Verabreichung eines LINK_TSG6-Polypeptids mehr als zweimal täglich umfasst, wobei die Behandlung gegebenenfalls die Verabreichung eines LINK_TSG6-Polypeptids weniger als viermal täglich umfasst.

5. LINK_TSG6-Polypeptid zur Verwendung nach Anspruch 3, wobei das LINK_TSG6-Polypeptid als Augentropfen formuliert ist.

6. LINK_TSG6-Polypeptid zur Verwendung nach einem der vorangegangenen Ansprüche, wobei LINK_TSG6 gemeinsam mit einem oder mehreren aus Prednisolon, Cyclosporin, Lifitegrast (Xiidra^{™}) oder künstlichen Tränen verabreicht wird.

7. LINK_TSG6-Polypeptid zur Verwendung nach einem der vorangegangenen Ansprüche, wobei etwa 10 µg bis 200 µg LINK_TSG6-Polypeptid pro Auge verabreicht werden, vorzugsweise 120 µg bis 150 µm LINK_TSG6-Polypeptid pro Auge verabreicht werden.

8. LINK_TSG6-Polypeptid zur Verwendung nach Anspruch 2, wobei die Unterdrückung einer Entzündung eine Verringerung der Produktion eines oder mehrerer proinflammatorischer Zytokine in der Hornhaut oder in intraorbitalen Tränendrüsen umfasst, wobei die proinflammatorischen Zytokine gegebenenfalls aus TNF-α, IL-6, IFN-γ und IL-1β ausgewählt sind.

9. LINK_TSG6-Polypeptid zur Verwendung nach Anspruch 2, wobei die Behandlung oder Prävention eines Syndroms des trockenen Auges eine Heilung von Hornhautepitheldefekten, eine Steigerung der Tränenproduktion, eine Unterdrückung einer Entzündung oder eine Erhöhung der Anzahl von Becherzellen in der Bindehaut im Vergleich zu Hornhautepitheldefekten, der Tränenproduktion, der Entzündung oder der Anzahl von Becherzellen in der Bindehaut vor der Verabreichung eines LINK_TSG6-Polypeptids umfasst.

10. LINK_TSG6-Polypeptid zur Verwendung nach Anspruch 2, wobei die Behandlung oder Prävention eines Syndroms des trockenen Auges eine Heilung von Hornhautepitheldefekten, eine Erhöhung der Tränenproduktion, eine Unterdrückung einer Entzündung oder eine Erhöhung der Anzahl von Becherzellen in der Bindehaut im Vergleich zu Hornhautepitheldefekten, der Tränenproduktion, der Entzündung oder der Anzahl von Becherzellen in der Bindehaut eines Kontrollindividuums aufweist, das mit einem TSG-6-Protein voller Länge behandelt wurde.

11. LINK_TSG6-Polypeptid zur Verwendung nach einem der vorangegangenen Ansprüche, wobei das behandelte Individuum am Sjögren-Syndrom leidet.

12. LINK_TSG6-Polypeptid zur Verwendung nach einem der vorangegangenen Ansprüche, wobei die Behandlung die Verabreichung von Augentropfen umfasst, die LINK_TSG6 umfassen.

13. Augentropfenformulierung, die ein LINK_TSG6-Polypeptid umfasst, wobei das LINK_TSG6-Polypeptid aus (i) der Aminosäuresequenz der SEQ ID NO: 7 oder SEQ ID NO: 9 oder (ii) einer Aminosäure mit einer Länge von bis zu 100 Aminosäuren und zumindest 90 % Identität mit der Aminosäuresequenz der SEQ ID NO: 7 oder SEQ ID NO: 9 besteht.

14. Augentropfenformulierung nach Anspruch 13, die zwischen 240 µg/ml und 3000 µg/ml, vorzugsweise zwischen 2400 µg/ml und 3000 µg/ml, LINK_TSG6-Polypeptid umfasst.

15. Augentropfenformulierung nach Anspruch 13 oder Anspruch 14, die weiters Predniso-Ion, Cyclosporin, Lifitegrast oder künstliche Tränen umfasst.

## Revendications

1. Polypeptide LINK_TSG6 pour utilisation dans le traitement ou la prévention d'un trouble de surface oculaire tel que la sécheresse oculaire, dans lequel le polypeptide LINK_TSG6 consiste en (i) la séquence d'acides aminés de SEQ ID NO :7 ou SEQ ID NO :9, ou (ii) une séquence d'acides aminés d'une longueur allant jusqu'à 100 acides aminés présentant au moins 90 % d'identité avec la séquence d'acides aminés de SEQ ID NO :7 ou SEQ ID NO :9.

2. Polypeptide LINK_TSG6 pour utilisation selon la revendication 1, dans lequel le traitement ou la prévention d'un trouble de surface oculaire tel que la sécheresse oculaire comprend un ou plusieurs effets choisis dans le groupe comprenant :
cicatrisation de défauts épithéliaux cornéens ;
augmentation de la production de larmes ; suppression d'inflammation ; et
nombre plus élevé de cellules caliciformes conjonctivales.

3. Polypeptide LINK_TSG6 pour utilisation selon la revendication 1 ou la revendication 2, dans lequel le traitement comprend une administration topique de polypeptide LINK_TSG6 à l'œil.

4. Polypeptide LINK_TSG6 pour utilisation selon l'une quelconque des revendications 1 à 3, dans lequel le traitement comprend une administration du polypeptide LINK_TSG6 deux fois par jour, facultativement dans lequel le traitement comprend une administration du polypeptide LINK_TSG6 plus de deux fois par jour, facultativement dans lequel le traitement comprend une administration du polypeptide LINK_TSG6 moins de quatre fois par jour.

5. Polypeptide LINK_TSG6 pour utilisation selon la revendication 3, dans lequel le polypeptide LINK_TSG6 est formulé sous la forme d'un collyre.

6. Polypeptide LINK_TSG6 pour utilisation selon l'une quelconque des revendications précédentes, dans lequel LINK_TSG6 est co-administré avec un ou plusieurs parmi la prednisolone, la cyclosporine, le Lifitegrast (Xiidra^{™}) ou des larmes artificielles.

7. Polypeptide LINK_TSG6 pour utilisation selon l'une quelconque des revendications précédentes, dans lequel environ 10 µg à 200 µg de polypeptide LINK_TSG6 sont administrés par œil, de préférence de 120 µg à 150 µg de polypeptide LINK_TSG6 sont administrés par œil.

8. Polypeptide LINK_TSG6 pour utilisation selon la revendication 2, dans lequel la suppression d'inflammation comprend une diminution de la production d'une ou plusieurs cytokines pro-inflammatoires dans la cornée ou les glandes lacrymales intra-orbitaires, les cytokines pro-inflammatoires étant facultativement sélectionnées parmi TNF-α, IL-6, IFN-γ et IL-1β.

9. Polypeptide LINK_TSG6 pour utilisation selon la revendication 2, dans lequel le traitement ou la prévention de la sécheresse oculaire comprend une cicatrisation de défauts épithéliaux cornéens, une augmentation de la production de larmes, une suppression d'inflammation ou une augmentation du nombre de cellules caliciformes conjonctivales par rapport aux défauts épithéliaux cornéens, une production de larmes, une inflammation ou un nombre de cellules caliciformes conjonctivales avant l'administration du polypeptide LINK_TSG6.

10. Polypeptide LINK_TSG6 pour utilisation selon la revendication 2, dans lequel le traitement ou la prévention de la sécheresse oculaire comprend la guérison de défauts de l'épithélium cornéen, l'augmentation de la production de larmes, la suppression de l'inflammation ou l'augmentation du nombre de cellules conjonctivales caliciformes par rapport aux défauts de l'épithélium cornéen, à la production de larmes, à l'inflammation ou au nombre de cellules conjonctivales caliciformes d'un individu témoin traité avec la protéine TSG-6 pleine longueur.

11. Polypeptide LINK_TSG6 pour utilisation selon l'une quelconque des revendications précédentes, dans lequel l'individu traité présente le syndrome de Sjögren.

12. Polypeptide LINK_TSG6 pour utilisation selon l'une quelconque des revendications précédentes, dans lequel le traitement comprend l'administration d'un collyre comprenant LINK_TSG6.

13. Formulation de collyre comprenant le polypeptide LINK_TSG6, dans laquelle le polypeptide LINK_TSG6 consiste en (i) la séquence d'acides aminés de SEQ ID NO :7 ou SEQ ID NO :9, ou (ii) une séquence d'acides aminés d'une longueur allant jusqu'à 100 acides aminés présentant au moins 90 % d'identité avec la séquence d'acides aminés de SEQ ID NO :7 ou SEQ ID NO **:9.**

14. Formulation de collyre selon la revendication 13, comprenant entre 240 µg/ml et 3 000 µg/ml, de préférence entre 2 400 µg/ml et 3 000 µg/ml de polypeptide_TSG6.

15. Formulation de collyre selon la revendication 13 ou la revendication 14, comprenant en outre de la prednisolone, de la cyclosporine, du Lifitegrast ou des larmes artificielles.
